(19) Europäisches Patentamt
European Patent Office
Office européen des brevets

(11) EP 3 802 849 B1

(12) EUROPEAN PATENT SPECIFICATION

(45) Date of publication and mention of the grant of the patent:
**19.07.2023 Bulletin 2023/29**

(21) Application number: **19726454.2**

(22) Date of filing: **29.05.2019**

(51) International Patent Classification (IPC):
***C12Q 1/18*** (2006.01) ***C12Q 1/04*** (2006.01)
***C12Q 1/02*** (2006.01) ***G01N 33/50*** (2006.01)

(52) Cooperative Patent Classification (CPC):
**C12Q 1/04; C12Q 1/02; C12Q 1/18; G01N 33/50**

(86) International application number:
**PCT/EP2019/064115**

(87) International publication number:
**WO 2019/229198 (05.12.2019 Gazette 2019/49)**

# (54) A FILM OF CHITOSAN AND A DEVICE COMPRISING THE SAME DEPOSITED ON A SUBSTRATE AND USES THEREOF

CHITOSANFILM UND VORRICHTUNG MIT DIESEM AUF EINEM SUBSTRAT UND DESSEN VERWENDUNG

FILM DE CHITOSANE ET DISPOSITIF LE COMPRENANT DÉPOSÉ SUR UN SUBSTRAT ET UTILISATIONS ASSOCIÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.05.2018 EP 18305666**

(43) Date of publication of application:
**14.04.2021 Bulletin 2021/15**

(73) Proprietors:
- **Centre national de la recherche scientifique 75016 Paris (FR)**
- **Université d'Aix Marseille 13007 Marseille (FR)**
- **Institut National de la Santé et de la Recherche Médicale 75013 Paris (FR)**
- **Institut National des Sciences Appliquées de Lyon 69621 Villeurbanne (FR)**
- **Université Jean Monnet Saint-Étienne 42100 Saint Étienne (FR)**
- **UNIVERSITE CLAUDE BERNARD - LYON 1 69100 Villeurbanne (FR)**

(72) Inventors:
- **MIGNOT, Tâm 13009 Marseille (FR)**
- **THEODOLY-LANNES, Olivier 13009 Marseille (FR)**
- **SUDRE, Guillaume 69004 Lyon (FR)**
- **DAVID, Laurent 69004 Lyon (FR)**
- **BUGNICOURT-MOREIRA, Loïc 69140 Rillieux-la-Pape (FR)**
- **TREGUIER, Julie 34090 Montpellier (FR)**

(74) Representative: **Cabinet Becker et Associés 25, rue Louis le Grand 75002 Paris (FR)**

(56) References cited:
**EP-B1- 1 246 650**
**WO-A1-2004/035767**
**WO-A2-2004/111188**
**WO-A2-2015/042583**
**US-A1- 2015 071 982**

- **FRANCES S. LIGLER ET AL: "Development of Uniform Chitosan Thin-Film Layers on Silicon Chips", LANGMUIR, vol. 17, no. 16, 1 August 2001 (2001-08-01), pages 5082-5084, XP055508487, US ISSN: 0743-7463, DOI: 10.1021/la010148b**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 802 849 B1

- Adrien Ducret ET AL: "Single cell microfluidic studies of bacterial motility", Methods in molecular biology, 31 January 2013 (2013-01-31), XP055508491, DOI: 10.1007/978-1-62703-245-2_6 Retrieved from the Internet: URL:https://www.researchgate.net/profile/Tam_Mignot/publication/234089367_Single_Cell_Microfluidic_Studies_of_Bacterial_Motility/links/55db210f08ae9d659492b41c/Single-Cell-Microfluidic-Studies-of-Bacterial-Motility.pdf?origin=publication_detail [retrieved on 2018-09-20]
- DIEGO I. CATTONI ET AL: "Super-Resolution Imaging of Bacteria in a Microfluidics Device", PLOS ONE, vol. 8, no. 10, 16 October 2013 (2013-10-16), page e76268, XP055507867, DOI: 10.1371/journal.pone.0076268
- YANYAN TANG ET AL: "Rapid Antibiotic Susceptibility Testing in a Microfluidic pH Sensor", ANALYTICAL CHEMISTRY, vol. 85, no. 5, 5 March 2013 (2013-03-05), pages 2787-2794, XP055268974, US ISSN: 0003-2700, DOI: 10.1021/ac303282j

## Description

### FIELD OF THE INVENTION

The present invention relates to a film of chitosan and its use for the adhesion of microorganisms, preferably bacteria, a device for microorganism growth comprising a substrate and a film of chitosan deposited thereon, and a method for preparing said device. The device of the invention is more particularly useful in methods for detecting and/or observing microorganisms and/or impacts thereon due to microenvironment changes, such as pH, medium, and/or bioactive agents. It can be used more specifically for *in vitro* determining the resistance or susceptibility of microorganisms to bioactive agents, said microorganisms being preferably bacteria. It can also be used in an *in vitro* method of screening for an antimicrobial agent of at least one microorganism or in a method for diagnosing a microbial infection in a patient.

### TECHNICAL BACKGROUND

In recent years, the development of methods for testing the impact of rapid and controlled environmental changes on microorganisms at the single cell level have sparked scientists' interest and the coupling of microfluidics with live-cell imaging has enabled a real breakthrough is this field. In addition, the advent of super-resolution microscopy has allowed microorganisms to be further explored at unprecedented resolution, tracking cellular processes one molecule at a time. The impact of these methods is not limited to basic research since single approaches are in particular powerful for determining antimicrobial susceptibility (AST, Antimicrobial or Antibiotic Susceptibility Testing) in record time: 1-2 hours to get the results of the test, while classic AST's require 8 to 24 hours.

However, a major technical bottleneck with the implementation of single cell approaches, especially for AST, is the immobilization of microorganisms. Agar surfaces have been widely used and promote the growth of a wide range of microorganisms, however such surfaces display several disadvantages such as: agar surfaces are not compatible with high-end microscopy (HEM) methods that requires cells to adhere to glass, and agar surfaces are also poorly compatible with experiments requiring rapid changes of the medium or injection of chemicals. Such experiments are generally performed by diffusion through the agar substrate but kinetics and exact concentrations are poorly controlled in such a device. Alternative methods have remedied these issues by growing bacteria immediately in contact with a glass surface. Because most bacteria do not adhere directly to glass, immobilization procedures are required, which include direct physical immobilization of the bacteria in micro-channels or glass functionalization by adhesive polymers. The use of micro-channels is certainly compatible with HEM and it allows AST in short times with high accuracy. However, this method has technical drawbacks: the use of microfluidic devices can be cumbersome, especially for routine commercial application, it can require extensive adaptation for a given bacterial species. Bacterial adhesion on glass can also be obtained by functionalizing a glass slide with adhesive polymers/molecules. However, this approach can also be difficult because the polymer has to be biocompatible and the surface chemistry for its functionalization can be complex.

Chen et al. (Anal. Chem. 2010, 82, 1012-1019) describes a method for determining the susceptibility of several *Escherichia coli* strains by means of a polydimethylsiloxane microfluidic channel system, with variable surface/volume ratios. Microscopic observations allow the determination of the minimum inhibitory concentration (MIC) and minimum bactericidal concentration (MBC). Results are obtained after about 2 hours, time from which the bacterial growth is modified in the presence of an antimicrobial agent. However, in practice, as specified above, handling such a microchannel system has proven to be difficult.

Dai et al. (Bioengineering, 2016, 3, 25) reviews uses of direct single-cell imaging on microfluidic devices for antibiotic susceptibility and toxicity testing. This strategy appears as a potential solution for fast AST, however bacteria need, in these methods, to be physically constrained. An alternative, reported by Chantell (Clin. Microbiol. Newsl. 2015, 37, 161-167), is the use of poly-L-lysine (PLL) to obtain a polycationic coated surface for immobilizing cells through coating-cell interactions. This approach remains slower since it is based on cell aggregates to form before their observation and not on the morphological changes at the single cell level. This aspect is a direct consequence of the toxicity of poly-L-lysine: indeed, poly-L-lysine dissipates proton motive force, imposing cells to divide out of the surface plan.

It is therefore important to work at developing new efficient coating materials on which cells can adhere and proliferate, without morphological alterations of the cells induced by the coating, allowing easy single-cell observations without physical constraints of the cells, and thus, enabling single cell AST studies.

Chitosan is a linear co-polysaccharide composed of randomly distributed D-glucosamine (GlcN) and N-acetyl-D-glucosamine (GlcNAc) units linked by β (1→4) glycosidic bonds. Chitosan can thus be represented by formula (I), as follows:

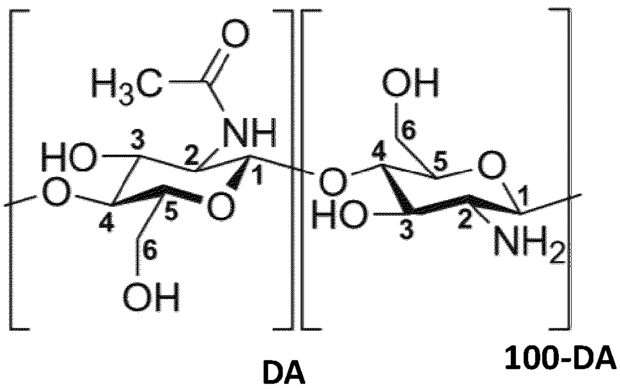

(I)

where, in formula (I), DA is the degree of acetylation, which can be defined as the percentage of the number of acetylated units, i.e. N-acetylglucosamine units, over the total number of units constituting the chitosan polymer, and 100 is the percentage of the total number of units constituting the chitosan polymer.

This polymer is commonly obtained by alkaline deacetylation of chitin, which can be found ubiquitously in nature, such as in insects or crustacean cuticles. A method for preparing a chitosan with a tailored degree of acetylation, starting from a chitinaceous material, is in particular described in WO 2004/11118. Chitosan thus represent a family of deacetylated derivatives of chitin with various degrees of acetylation (DA) and molecular masses, and has been widely studied, and many applications, in particular biomedical applications such as wound dressings (EP 1 246 650), have been envisioned with this material. Chitosan is known for its ability to not produce any adverse effects, when it is in contact with a biological system, and to be degraded by biological activity. Chitosan may also be used for its antimicrobial properties (Kong et al. Int. J. Food Microbiol., 2010, 144, 51-63). In particular, it was noticed that a low DA and a low pH tended to favor such properties (Carbohydr. Polym. 2017, 164, 268-283).

Actually, various properties and/or forms of chitosan materials such as gels, microparticles or films, may be obtained by adjusting physicochemical parameters involved in the preparation process (i.e. pH, concentration or ionic strength) in addition to its own structural features, in particular its degree of acetylation and its molecular mass. Methods for preparing a chitosan film and for coating such film on a surface have in particular been described in DE 202 15 659 and Ligler et al. (Langmuir 2001, 17, 5082-5084).

Ducret et al. (Methods Mol. Biol. 2013, 966, 97-107) describes a coating procedure for functionalizing a microfluidic chamber with chitosan, which enables adhesion and motility of the *Myxococcus xanthus* bacteria. In this study, a film of commercial chitosan is deposited on a transparent substrate and the bacteria establish contacts directly on the chitosan-treated coverslip. The contact zone is thus closer to the objective allowing advanced microscopy. However, commercial chitosan displays batch-to-batch variations and is rarely characterized by its DA and average molar mass although the biological and physico-chemical properties of chitosans are strongly impacted by these structural parameters (Schatz C. et al, Biomacromolecules, 2003, 4(3), 641). In that respect, commercial chitosan does not allow a satisfactory microorganism growth, preventing thereby from using such commercial chitosan for reliable assays. As described in WO 2015/042583, Cattoni et al. (PLOS One, 2013, 8, 10, e76268), and Tang et al. (Anal. Chem. 2013, 85, 2787-2794), various chitosan films have been used in microbial test devices, but are insufficiently characterized, in particular regarding the degree of acetylation.

## SUMMARY OF THE INVENTION

One goal of the present invention is to provide a chitosan material and a device comprising such chitosan material deposited on a substrate where said chitosan material allows an optimal adhesion and proliferation of microorganisms.

The Applicant has indeed identified a chitosan, with specific controlled degree of acetylation and molar mass, more specifically in a form of a film, which allows a good adhesion of microorganisms and do not affect their proliferation and their morphology.

The device of the invention enables an easy single-cell (or single-microorganism) observation that does not require a physical constraint of the cells (or microorganisms). Cells or microorganisms are merely interacting with the chitosan-coated substrate and adhere without covalent linkage, and multiple cell division cycles can be followed. The device is particularly well-suited for observing, detecting, or analyzing microorganisms and, to this end, the device may be associated with a simple microchannels system, that can be used to rapidly change the cell or microorganism's immediate environment and thus test for antimicrobial susceptibility testing or AST as well as the impact of other molecules

for other applications.

For AST, the invention provides a fast response, more specifically in less than 90 minutes.

An object of the invention is a chitosan material, characterized in that the chitosan has the following features:

- a molar mass between 100,000 and 900,000 g/mol, preferably between 150,000 and 610,000 g/mol, more preferably between 150,000 and 300,000 g/mol,
- a degree of acetylation between 32 and 57 %,

wherein said chitosan material is in the form of a film, preferably a film having a thickness between 5 and 500 nm, preferably between 40 and 220 nm.

Another object of the invention is a use of a chitosan material as defined above, as an *in vitro* adhesion material of microorganisms, preferably bacteria.

The invention also relates to a device for microorganism growth comprising a glass substrate, and a chitosan film deposited thereon, wherein said chitosan has the following features:

- a molar mass between 100,000 and 900,000 g/mol, preferably between 150,000 and 610,000 g/mol, more preferably between 150,000 and 300,000 g/mol, and
- a degree of acetylation between 25 and 65 %, preferably between 32 and 57 %.

The device according to the invention may be used for *in vitro* determining the resistance or susceptibility of microorganisms to bioactive agents. It can also be used in an *in vitro* method of screening for an antimicrobial agent of at least one microorganism or in an *in vitro* method for diagnosing a microbial infection in a patient.

The invention also relates to a method for preparing a device as defined herein, in which the chitosan film has a thickness between 5 and 500 nm, preferably between 40 and 250 nm, comprising the steps of:

(a) preparing a chitosan having the following features:

- a molar mass between 100,000 and 900,000 g/mol, preferably between 150,000 and 610,000 g/mol, more preferably between 150,000 and 300,000 g/mol,
- a degree of acetylation, between 25 and 65 %, preferably between 32 and 57 %;

(b) preparing an acidic aqueous solution of the chitosan prepared by step (a), which solution presents a chitosan concentration comprised between 0.1 and 3 % (w/w), preferably between 0.5 and 1.5 % (w/w);

(c) depositing the solution prepared in step (b) onto the substrate and forming a film of chitosan, preferably by spin coating, under conditions allowing to obtain a film thickness between 5 and 500 nm, preferably between 40 and 220 nm.

## BRIEF DESCRIPTION OF THE FIGURES

Abbreviations and nomenclature are specified in the Examples.

**Figures 1:**

**Figure 1A** shows the thickness of chitosan films (Shr) measured by ellipsometry according to chitosan (CS) concentration for various DA. The films were prepared from a chitosan acetate solution.

**Figure 1B** shows the thickness of chitosan films (Sqd) deposited by spin coating and measured by ellipsometry according to chitosan (CS) concentration for various DA. The films were prepared from a chitosan acetate solution.

**Figure 2:** shows the evolution of the contact angle measured on chitosan surfaces (Shr, DA 55 %, 0.67% w/w) with storage time at different temperatures.

**Figures 3:**

**Figure 3A** shows a device of the invention, which may be used for microorganisms observations.

**Figure 3B** shows the adhesion of Escherichia coli K12 on commercial chitosan without (left) or with (right) centrifugation (1000 rcf for 3 minutes).

**Figures 4:**

**Figure 4A** shows morphological anomalies obtained with a surface of chitosan with a low acetylation degree at different times.
**Figure 4B** shows the adhesion and division of Escherichia coli K12 on a chitosan $\chi$1 surface at different times.
**Figure 4C** shows a comparison of the adhesion of Escherichia coli K12 on glass and on chitosan $\chi$1 coated surface.
**Figure 4D** shows the behavior of Escherichia coli K12 on a poly-L-lysine coated surface at different times.

**Figures 5:**

**Figure 5A** shows the adhesion of Escherichia coli K12 on a chitosan $\chi$1 surface observed by transmission mode (Top) and RICM mode (bottom). In RICM, the cell substrate zone is dark when cell is adherent and bright when cell is non-adherent.
**Figure 5B** shows the adhesion of Escherichia coli K12 on a chitosan Ca 3% 0.67% surface observed by transmission mode (Top) and reflected interference contrast microscopy mode (or RICM mode) (bottom). In RICM mode, the cell substrate interface appears dark when the cell is adherent and bright when the cell is non-adherent, i.e. separated by a thin film of medium.
**Figure 5C** shows the statistical determination of generation time of Escherichia coli K12 on a chitosan $\chi$1 surface from individual morphological studies of n=257 bacteria.
**Figure 5D** shows Escherichia coli K12 cells after 72 hours on a chitosan $\chi$1 surface and the cell division restored after addition of fresh media.
**Figure 5E** shows the adhesion of Escherichia coli K12 according to cell cycle.

**Figures 6:**

**Figure 6A** shows the adhesion and division of Klebsiella pneumoniae ATCC 700603 on a chitosan $\chi$2 surface at different times.
**Figure 6B** shows the adhesion and division of Klebsiella pneumoniae LM21 on a chitosan $\chi$2 surface at different times.
**Figure 6C** shows the evolution of Klebsiella pneumoniae LM21 on a chitosan Sqd 35% 0.67% surface at different times.

**Figures 7:**

**Figure 7A** shows the effect of 100 mg/L of ampillicin on Escherichia coli K12.
**Figure 7B** shows dose-response of clinical strains from patients (named as UTI 227) for different concentrations of ETP.
**Figure 7C** shows the time to observe UTI 227 cell death according to ETP concentration.
**Figure 7D** shows cell width of clinical strains from patients (named as UTI 687) after 1 hour of incubation with different MEC concentrations.
**Figure 7E** shows growth results of UTI 227 with different ETP concentrations. Each line of the table is a different experiment. "+" : cell growth. "-" : bactericide/bacteriostatic effect of the antibiotic.

**Figure 8** shows the evolution of the growth rate of a bacterial strain according to the antibiotic concentration on a chitosan surface $\chi$1 (Curve A: UTI 704 as a bacterial strain and Mecillinam (MEC) as an antibiotic - Curve B: UTI 227 as a bacterial strain and Ertapenem (ETP) as an antibiotic).
**Figure 9** shows computationally-determined growth curves for strain UTI 227 with varying concentrations of ETP (number of detected cells across time with respect to ETP concentration). For each curve, the plot symbol is circular if the cells survive, and diamond shaped if the cell population stalls or shrinks due to cell death.
**Figure 10** shows an estimation of the minimal diagnostic time as determined automatically.
**Figures 11:**

**Figure 11A** shows Escherichia coli K12 stained by propidium iodide (50 $\mu$l/mL) after 30 seconds of incubation with and without EtOH 70%.
**Figure 11B** shows the evolution of UTI 687 cells stained by propidium iodide (50 $\mu$l/mL) in the presence of ETP (3mg/L).

## DETAILED DESCRIPTION OF THE INVENTION

The present invention is as defined in the appended claims.

The present invention thus relates to a chitosan material, characterized in that the chitosan has the following features:

- a molar mass between 100,000 and 900,000 g/mol, preferably between 150,000 and 610,000 g/mol, more preferably between 150,000 and 300,000 g/mol,
- a degree of acetylation between 32 and 57 %,

wherein said chitosan material is in the form of a film, preferably a film having a thickness between 5 and 500 nm, preferably between 40 and 220 nm. The chitosan film of the invention is advantageously water permeable.

Another object of the invention is a use of a chitosan material as defined above, for the *in vitro* adhesion of microorganisms or as an *in vitro* adhesion agent (or adhesion material) of microorganisms, preferably bacteria.

The invention also relates to a device comprising a glass substrate, and a chitosan film deposited thereon, wherein said chitosan has the following features:

- a molar mass between 100,000 and 900,000 g/mol, preferably between 150,000 and 610,000 g/mol, more preferably between 150,000 and 300,000 g/mol, and
- a degree of acetylation between 25 and 65 %, preferably between 32 and 57 %.

Said substrate (or support) of the device may be transparent or opaque. Said substrate may have any shape and any dimension.

In a particular embodiment, the substrate is a flat substrate. In another particular embodiment, the substrate is rigid, and more particularly flat and rigid.

In one preferred embodiment, the substrate of the device is a transparent substrate. According to the invention, a transparent substrate is a material which does not absorb a part of the light, selected wavelength(s) of the light, or does not absorb the light at all. Preferably, the transparent substrate is a glass slide or a glass coverslip. The transparent substrate according to the invention may be used as substrate for microscopic observations.

In a preferred embodiment, the glass substrate is a borosilicate glass substrate.

The device according to the invention comprises a glass substrate, coated with a chitosan film. Chitosan is typically obtained by chemical or enzymatic deacetylation of chitin. It can also be obtained by reacetylation of low DA chitosans. Chitin may be obtained from any sources in biomass, especially crustaceans shells, cephalopods endosqueletons, insects cuticles or cell walls of fungi. In particular, chitin may be obtained from shrimp shells or squids pens. Chitin and chitosan are also commercially available.

Chitosan may be defined by two main features: its degree of acetylation and its molar mass (or molecular weight or molecular mass). The degree of acetylation is the percentage of the number of acetylated units, i.e. N-acetylglucosamine units, over the total number of units constituting the chitosan polymer, i.e. N-acetylglucosamine and glucosamine units. The degree of acetylation may be experimentally determined by Nuclear Magnetic Spectroscopy (NMR) using Hirai method, as described by Hirai et al (Polym. Bull. 1991, 26, 87-94). The experimental degree of acetylation may be plus or minus 10 % of the theoretical degree of acetylation. For instance, a theoretical degree of acetylation of 55 % may lead to an experimental degree of acetylation comprised between 49,5 and 60,5 %.

The molar mass of chitosan may be dependent on the chitin source and on the degree of acetylation. For instance, a chitosan with a low degree of acetylation (DA<5%) is typically between 150,000 and 210,000 g/mol when obtained from shrimp chitin, and between 530,000 and 570,000 g/mol when obtained from squid chitin. A chitosan with a low molar mass can be obtained from chitosan with higher molar mass by carrying out any method known in the art (G.G. Allan, Carbohydrate Research 1995, 277(2), 273).

The chitosan according to the invention has a molar mass between 100,000 and 900,000 g/mol, preferably between 150,000 and 610,000 g/mol, and more preferably between 150,000 and 300,000 g/mol, and a degree of acetylation between 25 and 65 %, preferably between 32 and 57 %. Said chitosan may be obtained by a first deacetylation of chitin or chitosan to a DA typically below 5%, followed by a controlled re-acetylation. Said chitosan may also be obtained by controlled acetylation of a chitosan having a low degree of acetylation, for instance a degree of acetylation of 1% (such chitosan with low DA is commercially available). The deacetylation may be carried out by an enzyme, typically a chitin-deacetylase, or by a chemical agent, typically a base, such as sodium or potassium hydroxide. The acetylation or re-acetylation is controlled as to obtain the desired degree of acetylation as identified above and may be carried out using adjusted amounts of acetic anhydride, for instance in a chitosan hydroalcoholic solution. Typical procedures are described by Vachoud et al (Carbohydr. Res. 1997, 302, 169-177).

The dispersity (D), which is a measure of the distribution of molecular weights, also characterizes chitosan and

may also be dependent on the chitin source and on the degree of acetylation. Dispersity of a chitosan according to the invention is preferably between 1 and 3, more preferably between 1 and 2. Molar mass and dispersity may be determined by means of size exclusion chromatography (SEC), as described in M. Dumont et al., Carbohydrate Polymers 2018 190 31, or any other method.

According to specific embodiments, the chitosan of the invention is obtained from shrimp shells or squids pens.

According to specific embodiments, the chitosan of the invention has a molar mass from 120,000 to 220,000, or from 150,000 to 210,000, more particularly with a degree of acetylation of 55%.

According to specific embodiments, the chitosan of the invention has a molar mass from 530,000 and 610,000, more particularly with a degree of acetylation of 25% or 35%.

The device of the invention comprises a glass substrate and a chitosan film deposited thereon, wherein said chitosan has the above features.

More particularly, the glass substrate is directly coated with said chitosan film. In other words, no intermediate layer is thus deposited or present between the chitosan film and the substrate.

The present invention also provides a method for preparing such a device, in which the chitosan film has a thickness between 5 and 500 nm, preferably between 40 and 250 nm, comprising the steps of:

(a) preparing a chitosan having the following features:

- a molar mass between 100,000 and 900,000 g/mol, preferably between 150,000 and 610,000 g/mol, more preferably between 150,000 and 300,000 g/mol,
- a degree of acetylation between 25 and 65 %, preferably between 32 and 57 %;

(b) preparing an acidic aqueous solution of the chitosan prepared by step (a), which solution presents a chitosan concentration comprised between 0.1 and 3 % (w/w), preferably between 0.5 and 1.5 % (w/w);

(c) depositing the solution prepared in step (b) onto the glass substrate, and forming a film of chitosan, preferably by spin coating, under conditions allowing to obtain a film thickness between 5 and 500 nm, preferably between 40 and 220 nm.

After preparing a chitosan with the above identified features (step (a)), said chitosan is solubilized in an acidic aqueous solution. The acidic aqueous solution is typically a solution of at least one acid in a solvent, such as water. Preferably, water is deionized water. Said at least one acid of the acidic aqueous solution may be any organic acid, inorganic acid or a mixture thereof. Said at least one acid may be hydrochloric acid, hydrobromic acid, hydriodic acid, hydrofluoric acid, nitric acid, sulfuric acid, hexafluorophosphoric acid, tetrafluoroboric acid, trifluoroacetic acid, acetic acid, sulfonic acid such as methanesulfonic acid, mono- or polycarboxylic acid, or mixtures thereof. Preferably, said at least one acid is acetic acid or hydrochloric acid. The concentration of said at least one acid in the acidic aqueous solution may be adjusted so that the ratio (H+:NH2) of the amount of acid-equivalent of said at least one acid in the acidic aqueous solution to the amount of amine-equivalent groups of chitosan, i.e. non-acetylated amine groups, of the chitosan in the acidic aqueous solution is between 1.4:1 and 0.8:1, preferably 1:1. This ratio aims at avoiding or limiting acid hydrolysis of the glyosidic linkages. In the method according to the invention, the concentration of chitosan in the acidic aqueous solution is comprised between 0.1 and 3 % (w/w), preferably between 0.5 and 1.5 % (w/w). Preferably, the pH of the chitosan acidic aqueous solution ranges from 4 to 5.

"Acid-equivalent" refers to the actual number of protons that can be released by a given acid. For instance, one mole of sulfuric acid (H2SO4) can release two moles of acid-equivalent.

In the method according to the invention, the solution of chitosan thus obtained is then deposited on a glass substrate, and a film of chitosan is formed. Said film has preferably a thickness comprised between 5 and 500 nm, preferably between 40 and 250 nm.

The thickness can typically be measured by any method, preferably by ellipsometry profilometry or Atomic Force Microscopy (AFM).

Said film may be formed or prepared by means of any coating process, such as for instance dip-coating or spin-coating. Preferably, said film of chitosan is formed by spin-coating. Spin-coating is a process in which a small amount of a solution comprising a coating material is deposited on the center of the substrate. The rotation of the substrate at high speed, by means of a spin-coater, spreads the coating material by centrifugal force, evaporates the solvent and allows to obtain a thin film of material onto the substrate.

In order to get a thickness as defined above, the speed and the duration of the rotation can be adjusted to any suitable range, depending on:

- the viscosity of the chitosan solution, which itself depends on the concentration of the chitosan solution and the structural features of chitosan,

- and the size of the substrate.

In a particular embodiment, the surface area of said chitosan film is comprised between 10 $\mu m^2$ and 80 $cm^2$, preferably between 50 $\mu m^2$ and 10 $cm^2$. In a preferred embodiment, the surface area of the substrate is the same as the surface area of the chitosan film.

In a particular embodiment, the device of the invention further comprises a channels system, more specifically a microfluidic channels system, set onto the chitosan film. Preferably, said channels system is a microchannels system, set onto the chitosan film. Said channels system may be stuck to the chitosan-coated surface of the device of the invention, for instance by means of an adhesive tape. Said channels system may be made of any material, such as glass, plastic, or polydimethylsiloxane (PDMS). Said channels system may comprise one or several channels, and each channel may be connected at both ends to a well. One of the two wells may be used as a channel input, and the other one may be used as a channel output.

Said channels system is preferably bottomless, so that the chitosan-coated surface plays the role of the bottom when said channels system is set thereon.

The channels system may present variable surface/volume ratios. Surfaces and volumes thereof can be adapted by one skilled in the art depending on the intended use. More specifically, each channel of said channels system may independently have a volume ranging from 400 to 10 $\mu L$. Said channels system may be connected to one or more syringes optionally automatically pushed by a push-syringe.

The chitosan material film of the invention is particularly useful or suitable, preferably under *in vitro* conditions, for the adhesion of microorganisms, preferably bacteria. The device with chitosan film according to the invention is advantageously water-permeable. Also, said device which is suitable for microorganism growth advantageously enables microorganisms to adhere and proliferate on its surface.

The device of the invention advantageously allows an easy single-cell or single-microorganism observation that does not require physical constraints of the cells or microorganisms. Cells or microorganisms can adhere without covalent linkage, and several cells divisions can thus be advantageously followed. Therefore, the device according to the invention may be advantageously used, preferably under *in vitro* conditions, for detecting and/or observing microorganisms and/or biological processes involving microorganisms and/or impacts thereon due to microenvironment changes, such as pH, medium, and/or bioactive agent.

For this purpose, the device may be associated with any suitable type of microscopy, such as optical or electron microscopy after fixation. Examples of microscopy include transmission electron microscopy (TEM), scanning electron microscopy (SEM), atomic force microscopy (AFM), bright field microscopy, dark field microscopy, phase contrast microscopy, interference reflection microscopy such as Reflection Interference Contrast Microscopy (RICM), fluorescence microscopy, confocal microscopy, high-end microscopy or super-resolution microscopy such as Structured Illumination Microscopy (SIM), Photoactivated Light Microscopy (PALM and STORM) and stimulated emission depletion microscopy (STED). Preferably, the device is associated with optical microscopy. Any mode of microscopy such as light-sheet, Wet-SEEC or Raman, may be used with said optical microscopy.

Said device may be used, preferably under *in vitro* conditions, for detecting microorganisms, observing microorganisms, or more specifically detecting growth thereof.

According to an embodiment, the chitosan film and the device comprising the same can be used in an *in vitro* method for determining the resistance or susceptibility of microorganisms to bioactive agents. According to another embodiment, the chitosan film and the device comprising the same can be used in an *in vitro* method of screening for an antimicrobial agent of at least one microorganism. According to another embodiment, the chitosan film and the device comprising the same can be used in a method, preferably an *in vitro* method, for diagnosing a microbial infection in a patient.

The microorganisms are preferably bacteria. For the uses and methods as identified above, the device is preferably associated with any suitable type of microscopy, such as optical or electron microscopy.

Microorganisms are typically members of one of following classes: bacteria, fungi, algae, oomycetes and protozoa, and which can for purposes of the present invention include viruses, prions or other pathogens. In one aspect, bacteria, and in particular human, animal and vegetal bacteria pathogens are evaluated. Suitable microorganisms include any of those well established in biology and those novel pathogens and variants that emerge from time to time. Examples of currently known bacterial pathogens, for example, include, but are not limited to, genera such as Bacillus, Vibrio, Escherichia, Shigella, Salmonella, Mycobacterium, Clostridium, Cornyebacterium, Streptococcus, Staphylococcus, Haemophilus, Neisseria, Yersinia, Plesiomonas, Pseudomonas, Chlamydia, Bordetella, Treponema, Stenotrophomonas, Acinetobacter, Enterobacter, Klebsiella, Proteus, Serratia, Citrobacter, Enterococcus, Legionella, Mycoplasma, Chlamydophila, Moraxella or Morganella,. Similarly, microorganisms may comprise fungi selected from a set of genera such as Candida or Aspergillus. Still other microorganisms may comprise pathogenic viruses , including, but not limited to, orthomyxoviruses, (e.g. influenza virus), paramyxoviruses (e.g. respiratory syncytial virus, mumps virus, measles virus), adenoviruses, rhinoviruses, coronaviruses, reoviruses, togaviruses (e.g. rubella virus), parvoviruses, poxviruses (e.g.

variola virus, vaccinia virus), enteroviruses (e.g. poliovirus, coxsackievirus), hepatitis viruses (including A, B and C), herpesviruses (e.g. Herpes simplex virus, varicella-zoster virus, cytomegalovirus, Epstein-Barr virus), rotaviruses, Norwalk viruses, hantavirus, arenavirus, rhabdovirus (e.g. rabies virus), retroviruses (including HIV, HTLVI and II), papovaviruses (e.g. papillomavirus), polyomaviruses, picornaviruses, or the like. In the viral aspect, in general, the methods and compositions of the invention may be used to identify host cells harboring viruses.

In a preferred embodiment, microorganisms are bacteria. In a more preferred embodiment, microorganisms are enterobacteria. Examples of enterobacteria include, but are not limited to, Citrobacter, Enterobacter, Escherichia, Klebsiella, Morganella, Pectobacterium, Plesiomonas, Proteus, Salmonella, Shigella, Serratia or Yersinia. Preferred examples are Klebsiella and Escherichia. Preferably, Klebsiella is Klebsiella pneumoniae and Escherichia is Escherichia coli.

According to specific embodiments, the chitosan used according to the invention has a molar mass from 120,000 (or 150,000) to 210,000, more particularly with a degree of acetylation of 55%, and is more particularly useful for detecting, observing microorganisms or growth thereof, where the microorganisms are more specifically Klebsiella (e.g. Klebsiella pneumoniae) or Escherichia (e.g. Escherichia coli).

Said bacteria may come from a bacteria culture, such as a primoculture or a blood culture, and/or a body fluid, such as urine.

Bioactive agents may be any compound which can modulate the growth of microorganisms or can have any biological impact on the growth of the studied microorganisms. Bioactive agents can therefore include any type of compounds that can modulate the microenvironment of the microorganisms, such as pH or medium. They include, but are not limited to, drugs, hormones, proteins, nucleic acids, or any organic or inorganic chemical compounds, for instance enzymatic substrates that can be assimilated by the microorganisms.

Bioactive agents are typically antimicrobial agents (or antimicroorganism agents), which include, but are not limited to, antibiotic families (bactericides or bacteriostatic compounds), such as cephalosporins, penicillins (such as ampicillin or mecillinam), carbapenems (such as ertapenem), monobactams, other novel beta-lactam antibiotics , beta-lactamase inhibitors, quinolones (such as ofloxacin), fluoroquinolones, macrolides, ketolides, glycopeptides, aminoglycosides, fluoroquinolones, ansamycins, azalides, lincosamides, lipopeptides, glycolipopeptides, streptogramins, polymyxins, tetracyclines, phenicols, oxazolidinones, nitroamidazoles, folate pathway inhibitors, and other families, as well as bacteriophages, including novel agents, used as antibiotics in clinical practice or in research. Antiviral agents are also included within the definition of antimicrobial agents and include both known approved antiviral agents as well as experimental ones. In addition, combinations of these agents can be tested, particularly in light of the evolution of resistant strains. Also, as described herein, the concentration of the antimicroorganism agent may be changed over time to reflect the pharmacokinetics of the antimicroorganism agent in biological tissues.

Preferably, the bioactive agent is an antimicrobial agent, and more specifically an antibiotic. Preferred antibiotics are ampicillin, ofloxacin, ertapenem, mecillinam or a mixture thereof.

According to specific embodiments, the chitosan used according to the invention has a molar mass from 150,000 and 210,000, more particularly with a degree of acetylation of 55%, and the antibiotics used in the methods according to the invention are more preferably ampicillin, ofloxacin, ertapenem, mecillinam or a mixture thereof.

The invention also provides methods, preferably *in vitro* methods, for detecting microorganisms, observing microorganisms and/or determining the resistance or susceptibility of microorganisms to bioactive agents, by means of the device according to the invention, preferably associated with any suitable type of microscopy, such as those mentioned above. The detection and/or observation may be done at the individual or discrete microorganism level, rather than at the colony level. Therefore, detecting growth or alterations in growth of individual microorganisms and/or observing at least one microorganism may be done as an evaluation of an individual cell in a period of time such that a small population of daughter cells can be formed, and therefore preferably prior to the ability to visually see a colony with the naked eye.

The methods of the invention can be applied to the response of microorganisms to bioactive agents, including but not limited to, proteins, hormones, drugs, for instance for drug sensitivity testing, or environmental agents. These agents can be so analyzed, as long as the response is detectable by the detector employed. In some cases, a stain or any kind of label may be required in order to make the response to the condition visible or at least, visible at an earlier stage.

Growth may refer to positive growth, a lack of growth, e.g. cells that are not actively dividing but are not growing positively, and negative growth, e.g. death.

"Positive growth" in the case of microorganisms that are cells (e.g. bacteria, protozoa and fungi) refers to the increase in size and/or procession of cell division, and particularly includes the production of daughter cells. Thus, "detecting positive growth" of a discrete microorganism refers to detecting either an increase in the size of the microorganism and/or detecting the presence of cell division, which may or may not increase the total area occupied by the microorganism. It should be noted that in some cases, positive growth can be detected as an increase in the area on the detection surface that the parent cell or daughter cells occupy. In the case of viruses, "positive growth" refers to the reproduction of viruses, generally within a host cell, and can include host cell lysis, in the case of lytic viruses. Thus the "positive growth" of a virus may sometimes be detected as a loss of the discrete host cell. "Detecting growth" can also

refer to detecting a lack of growth, e.g. either neutral or negative growth. That is, some antimicrobial agents act by retarding positive growth yet do not kill the cells; this is generally referred to a "neutral growth". Thus, detecting little or no change in the size, shape, volume and/or area of a cell (on a surface) is included within the evaluation of "growth", e.g. in the absence of an agent, a microorganism will exhibit positive growth, but in the presence of the agent, a lack of growth is significant, even if the microorganism does not die. It should be noted that in some cases, there will be small changes in the size, shape, volume and/or area of a cell on the detection surface, but this can be distinguished from positive growth. "Detecting growth" can also refer to detecting negative growth, e.g. necrosis. In addition, there have been some limited discussions of bacterial programmed cell death (e.g. apoptosis and/or autophagic cell death), which would be considered negative growth as well. In general, detecting negative growth relies on changes, usually but not always decreases, in microorganism size, shape, area or volume that can be detected by the methods of the invention.

Thus, "detecting growth" or "detecting alterations in growth" can refer to detecting positive growth, a lack of growth, e.g. detecting cells that are not actively dividing but are not growing positively, and negative growth, e.g. death. In general, the invention is unique in that detecting growth is done at the individual or discrete microorganism level, rather than at the colony level. "Detecting growth" or "detecting alterations in growth" can be carried out by measurement of minimum inhibitory concentration (MIC), minimum bactericidal concentration (MBC), time-kill kinetics, suppression of cell division, resistance induction and selection, and/or pharmacodynamic parameters.

"Detecting growth of an individual microorganism" is done as an evaluation of growth of an individual cell in a period of time such that a small population of daughter cells can be formed, but prior to the ability to visually see a colony with the naked eye. Thus, the "quantum microbiology" component of the invention allows detection within a single doubling time of a microorganism, rather than tens or hundreds of doubling times. In addition, the methods of the invention do not necessarily require an initial growth of microorganisms (either liquid or solid) prior to the assay; the present invention is sensitive enough to start with biological samples with no growth prior to the assay.

Labels may also be used for the detection and/or observation of microorganisms. Suitable labels include, but are not limited to, enzyme indicators, optical labels, such as optical dyes, fluorescent dyes, quantum dots, colored particles or phase contrast particles, chemiluminescent indicators or electrochemical indicators.

Assays can be carried out to test antimicrobial agents and their efficacy, such assays can include measurement of minimum inhibitory concentration (MIC), minimum bactericidal concentration (MBC), time-kill kinetics, suppression of cell division, resistance induction and selection, and/or pharmacodynamic parameters.

## Assays

As mentioned above, the invention relates to an *in vitro* use of the chitosan material as defined above, and more preferably a chitosan film as defined above, for the *in vitro* adhesion of microorganisms or as an *in vitro* adhesion agent (or adhesion material) of microorganisms, preferably bacteria.

In that respect, the chitosan material and the device of the invention can be a useful tool for assays in order to detect growth of microorganisms, allowing thereby determination of resistance or susceptibility of microorganisms to bioactive agents, identification of antimicrobial agent for at least one microorganism or diagnosis/detection of a microbial infection in a patient.

One assay can lie in the incubation of the microorganism in a constant concentration of antimicrobial agent. An increased number of the microorganism (positive growth) after a period of incubation indicates an absence of susceptibility of the microorganism to the antimicrobial agent at the concentration used, whereas a neutral or negative growth indicates a susceptibility of the microorganism to the microbial agent at the concentration used. Higher concentrations may be used until causing a neutral or negative growth. By increasing the amount of antimicrobial agent in steps over a period of time, or by using different concentrations in different channels at the same time, a minimum inhibitory concentration (MIC) can be determined. In addition, because viability of the microorganism can also be determined at each concentration, a minimum bactericidal concentration (MBC) can also be determined.

The detection of growth (including viability) at different concentrations of antimicrobial agent in a medium can be performed either by using a channels system, each of which challenges the microorganism with a specific concentration of antimicrobial agent, or alternatively, by increasing the concentration within a given channel of the channels system. In the former case, the time response of the microorganisms can be easily established, as well as the persistent response of the microorganism once the antimicrobial agent has been removed (e.g. a post-antibiotic effect). That is, the microorganism can also be challenged with a given concentration of antimicrobial agent for a brief period, and then the medium replaced with a medium lacking the antimicrobial agent, and the growth of the microorganisms can be monitored over time.

In a first embodiment, the invention provides a method, preferably an *in vitro* method, for determining the resistance or susceptibility of microorganisms to bioactive agents, comprising the steps of:

(a) contacting a sample containing microorganisms with the chitosan film of said device;
(b) maintaining said microorganisms in contact with the film for a first period of time;

(c) adding a solution comprising at least one bioactive agent;
(d) maintaining said microorganisms and solution in contact with the film for a second period of time;
(e) detecting growth of individual microorganisms after the addition of said bioactive agent, more specifically by microscopy.

In another embodiment, the assays herein can be used to test candidate antimicrobial agents and their efficacy, including measurement of MIC, MBC, time-kill kinetics, suppression of cell division, resistance induction and selection, and pharmacodynamic parameters. Accordingly, the invention provides a method, preferably an *in vitro* method, of screening for an antimicrobial agent of at least one microorganism, comprising the steps of:

(a) contacting a sample containing at least one microorganism with the chitosan film of said device;
(b) maintaining said at least one microorganism in contact with the film for a first period of time;
(c) adding a solution comprising at least one candidate antimicrobial agent;
(d) maintaining said at least one microorganism and solution in contact with the film for a second period of time;
(e) detecting growth of individual microorganisms after the addition of said candidate agent, more specifically by microscopy; and
(f) identifying the candidate as an antimicrobial agent on the basis of susceptibility of the said at least one microorganism to said candidate agent, as compared to a negative control.

In yet another embodiment, the invention provides a method, preferably an *in vitro* method, for diagnosing a microbial infection in a patient, comprising the steps of:

(a) contacting a sample from the patient with the chitosan film of said device;
(b) maintaining said sample in contact with the film for a first period of time;
(c) optionally adding a solution comprising at least one bioactive agent and maintaining said sample and solution in contact with the film for a second period of time; and,
(d) detecting growth of individual microorganisms from said sample, more specifically by microscopy, as an indication of the presence of microorganisms in the sample.

According to said last embodiment, the sample may or may not contain microorganisms, the main purpose of said method being to detect or not the presence of microorganisms in a sample from patients.

<u>Steps (a) and (b) in the methods of the invention</u>

In said methods, the sample possibly containing microorganisms may be any environmental, clinical, human, animal or other sample. In case of diagnosis, the patient can be any human or non-human mammal. The sample can be used as such or after culture thereof by any well-known method. The sample can thus be a microorganism culture (preferably a bacteria culture), such as a primoculture, a blood culture, a subculture thereof, and/or a body fluid, such as urine. In methods of the invention, said sample, and in particular said human or non-human sample, is an *in vitro* sample, namely a sample collected from said human or non-human, and used according to said methods outside its normal biological context.

The microorganisms contained in said sample may be at any stage of the cell cycle. The microorganisms contained in said sample may be in an exponential phase or in a stationary phase. The Optical Density of said sample can be comprised between 0.1 and 1, preferably between 0.4 and 0.6.

In one embodiment, the sample containing microorganisms is not diluted prior to contact with the chitosan film of the device of the invention. In another embodiment, the sample containing microorganisms is diluted prior to contact with the chitosan film of the device of the invention.

The dilution may be carried out in a medium suited for microorganisms, known by the skilled artisan, such as a Luria-Bertani (LB) medium. Such a medium may include, but is not limited to, water, yeast extracts, and any salt, such as MgCl2, CaCl2, ZnCl2, KCl or a combination thereof, such salts being among many others sources of Mg, Ca, Zn and/or K. Such a medium may be at a proper temperature and oxygen level (such as saturation or depletion). Said sample can be diluted such that the microorganisms contained therein or growth thereof can be detected or identified. The Optical Density after dilution may be adjusted by the skilled artisan, according to the implemented conditions of the method, for instance depending on the nature of the microorganism studied.

In a particular embodiment, the device of the invention comprises a channels system, and the sample used in the methods of the invention is introduced in one or more channels through one or more corresponding wells of the channels system, optionally by means of a syringe..

Once the sample optionally containing microorganisms is contacted with the chitosan film of a device of the

invention (step (a)), sample or microorganisms are subjected to a first period of time (step (b)), i.e. samples or microorganisms are maintained in contact with the film for a first period of time. Said first period of time typically allows adhesion of microorganisms to the chitosan film. Microorganisms may be growing during said first period of time. In one embodiment, said first period of time includes a period of sedimentation of the microorganisms, which may be comprised between 1 minute and 2 hours, preferably between 15 and 30 minutes. In another embodiment, said first period of time includes a centrifugation. Centrifugation may be carried out for 10 seconds to 20 minutes, preferably 2 to 5 minutes, at 10 to 5000 rcf (i.e. relative centrifugal force), preferably 100 to 1500 rcf.

Said first period of time may end up with a rinse step with an appropriate medium in order to remove non-adherent microorganisms.

The device of the invention allows a good adhesion of microorganisms. Adherent microorganisms may typically resist to a shear stress lower or equal to 1.5 Pa.

The system obtained at the end of step (b) may be the same for anyone of the methods of the invention.

Determining the resistance/susceptibility of microorganisms to bioactive agents:

In another embodiment, a method of the invention is a method, preferably an *in vitro* method, for determining the resistance or susceptibility of microorganisms to bioactive agents, which comprises a step (c) consisting in adding a solution comprising at least one bioactive agent. The solution is thus added to the sample in contact with the film. Said solution comprising at least one bioactive agent may be an aqueous solution. In a preferred embodiment, said solution comprising at least one bioactive agent is a medium suited for microorganisms, such as the Luria-Bertani (LB) medium described above. The concentration of said at least one bioactive agent may vary in a large extent. It can be comprised from 0 (as a control) to 500 mg/L. Said bioactive agent is preferably an antimicrobial agent, and more preferably an antibiotic.

In a particular embodiment, a "solution containing at least one bioactive agent" refers to several solutions containing one or more bioactive agents, each solution being added successively or simultaneously, and each bioactive agent being independently identical or different. Generally, a plurality of assay mixtures are run in parallel with different agent concentrations to obtain a differential response to the various concentrations. Typically, one of these concentrations serves as a negative control, i.e., at zero concentration or below the level of detection.

In a particular embodiment, the device of the invention comprises a channels system, and said solution comprising at least one bioactive agent is introduced in one or more channels through one or more corresponding wells of the channels system, optionally by means of a syringe. By the use of a channels system, comparisons between different bioactive agents or samples, or different concentrations thereof, can be made, for instance to detect alterations in growth of individual microorganisms.

In step (d), said microorganisms and solution are subjected to a second period of time. In other words, they are maintained in contact with the film for a second period of time. Said second period of time may be comprised between 0 and 24 hours, preferably 10 and 120 minutes. Said second period of time may be dependent on various parameters which include, but are not limited to, said microorganisms or said at least one bioactive agent of the solution.

Said second period of time can include a period of growth of microorganisms. Growth may refer to positive growth, a lack of growth, e.g. cells that are not actively dividing but are not growing positively, and negative growth, e.g. death. In particular, alterations in growth, which includes cell death, may occur due to the addition of the bioactive agent(s), and may thus be detected in step (e), by means of any type of microscopy, such as those mentioned above.

Steps (d) and (e) may be carried out successively or simultaneously. Steps (e) and (f) may be carried out successively or simultaneously.

The resistance or susceptibility of microorganisms to at least one bioactive agent may be assessed by step (e) through the determination of the "minimum inhibitory concentration" or "MIC", and/or the study of a dose-dependent response, thus revealing the efficacy or not of said at least one bioactive agent.

According to the method of the invention, the determination of the resistance or susceptibility of microorganisms to at least one bioactive agent can advantageously require less than 180 minutes, preferably less than 90 minutes, more preferably less than 60 minutes after adding the solution containing at least one bioactive agent. Thanks to the device of the invention, a fast antibiotic susceptibility testing can be provided.

In a particular embodiment, the solution comprising at least one bioactive agent added in step (c) further comprises a DNA stain, such as propidium iodide. The use of a DNA stain may facilitate microscopic observations, by crossing selectively the membrane of dead or dying cells, and thus, by selectively staining these dead or dying cells.

Screening for an antimicrobial agent of at least one microorganism

In another embodiment, a method of the invention is an *in vitro* method for screening for an antimicrobial agent of at least one microorganism, which comprises a step (c) consisting in adding a solution comprising at least one candidate

antimicrobial agent. The solution comprising at least one candidate antimicrobial agent is thus added to the sample in contact with the film. Said solution comprising at least one candidate antimicrobial agent may be an aqueous solution. In a preferred embodiment, said solution comprising at least one candidate antimicrobial agent is a medium suited for microorganisms, such as the Luria-Bertani (LB) medium described above.

By "candidate antimicrobial agent" as used herein describes any compound, e.g., protein, nucleic acid, small organic molecule, polysaccharide, etc. that can be screened for activity as outlined herein. Generally, a plurality of assay mixtures are run in parallel with different agent concentrations to obtain a differential response to the various concentrations. Typically, one of these concentrations serves as a negative control, i.e., at zero concentration or below the level of detection.

In a particular embodiment, the device of the invention comprises a channels system, and said solution comprising at least one candidate antimicrobial agent is introduced in one or more channels through one or more corresponding wells of the channels system, optionally by means of a syringe. By the use of a channels system, comparisons between different bioactive agents or samples, or different concentrations thereof, can be made to detect alterations in growth of individual microorganisms.

In step (d), said microorganisms are subjected to a second period of time. In other words, they are maintained in contact with the film for a second period of time. Said second period of time may be comprised between 0 and 48 hours, preferably 20 minutes and 2 hours. Said second period of time may be dependent on various parameters which include, but are not limited to, said microorganisms or said at least one candidate agent.

Said second period of time can include a period of growth of microorganisms. Growth may refer to positive growth, a lack of growth, e.g. cells that are not actively dividing but are not growing positively, and negative growth, e.g. death. In particular, alterations in growth, which includes cell death, may occur due to the addition of the candidate agent(s), and may thus be detected in step (e), by means of any type of microscopy, such as those mentioned above. Alterations in growth may be advantageously detected in less than 180 minutes, preferably in less than 60 minutes, after adding the at least one candidate agent.

Steps (d) and (e) may be carried out successively or simultaneously. Steps (e) and (f) may be carried out successively or simultaneously.

In a particular embodiment, the solution comprising at least one chemical or bioactive agent added in step (d) further comprises a DNA stain, such as propidium iodide. The use of a DNA stain may facilitate microscopic observations, by crossing selectively the membrane of dead or dying cells, and thus, by selectively staining these dead or dying cells.

Diagnosis of microbial infection

In one embodiment, a method of the invention is a method, preferably an *in vitro* method, for diagnosing a microbial infection in a patient, which comprises an optional step (c) consisting in adding a solution comprising at least one bioactive agent and maintaining the sample and solution in contact with the film for second period of time. According to step (c), the solution comprising at least one bioactive agent is thus added to the sample in contact with the film. Said solution comprising at least one bioactive agent may be an aqueous solution. In a preferred embodiment, said solution comprising at least one bioactive agent is a medium suited for microorganisms, such as the Luria-Bertani (LB) medium described above. It can also be a solution comprising enzymatic substrates, as bioactive agents, which are known to be assimilated by specific microorganisms. According to such embodiment, the enzymatic substrates can be labelled and detection of growth of individual microorganisms can thus be followed by the labelling of microorganisms. Labelling of specific microorganisms is an indication of the presence of such specific microorganisms in the sample and microbial infection of the patient can thus be diagnosed.

In a particular embodiment, a "solution containing at least one bioactive agent" refers to several solutions containing one or more bioactive agents, each solution being added successively or simultaneously, and each bioactive agent being independently identical or different.

In another particular embodiment, the device of the invention comprises a channels system, and said solution comprising at least one bioactive agent is introduced in one or more channels through one or more corresponding wells of the channels system, optionally by means of a syringe. By the use of a channels system, comparisons between different bioactive agents or samples, or different concentrations thereof, can be made to detect alterations in growth of individual microorganisms.

Said second period of time may be comprised between 0 and 48 hours, preferably 10 minutes and 2 hours. Said second period of time may be dependent on various parameters which include, but are not limited to, said microorganisms or said at least one bioactive agent.

Said second period of time advantageously includes a period of growth of microorganisms. Growth of individual microorganisms may be finally detected by means of any suitable type of microscopy, such as those mentioned above. Growth may refer to positive growth, a lack of growth, e.g. cells that are not actively dividing but are not growing positively, and negative growth, e.g. death.

Steps (c) and (d) may be carried out successively or simultaneously.

This invention will be better understood in light of the following examples which are given for illustrative purposes only and do not intend to limit the scope of the invention, which is defined by the attached claims.

## Abbreviations

In the following examples, chitosan films may be described as follows : "*Source DA [c]"*, wherein *Source* is the source of chitin used to prepare the chitosan, i.e. Shr (Shrimp) or Sqd (Squid) ;

*DA* is the Degree of Acetylation of the chitosan ;
*[c]* is the Concentration (w/w) of the chitosan solution used to form the chitosan film.

Example: "Sqd 55% 0.67%" refers to a chitosan film which chitin source is squid, and having a degree of acetylation of 55% and prepared with a chitosan solution of concentration 0.67%w/w.

OD stands for Optical Density.

More specifically, $\chi$1 refers to "Shr 55% 0.67%" and $\chi$2 refers to "Shr 55% 1.3%".

WT stands for "Wild Type".

ESBL stands for "Extended-Spectrum Beta-Lactamases".

ETP stands for Ertapenem. MEC stands for mecillinam. OFLO stands for ofloxacin. AMP stands for ampicillin.

UTI stands for Urinary Tract Infection. UTI followed by a number (such as UTI 227 or UTI 687) refers to an Escherichia coli strain from an Urinary Tract Infection.

## EXAMPLES

### 1-Preparation and characterization of chitosan material and chitosan-coated surfaces.

#### Materials

Chitosan with low degree of acetylation (DA) and different molar mass ($M_w$) ((a) DA 1.0 $\pm$ 0.5%; $M_w$ = 156.1 kg/mol, Đ = 1.8 (Shr) and (b) DA 2.4 $\pm$ 0.5%; 557.2 kg/mol, Đ = 1.4 (Sqd)) were purchased from Mahtani Chitosan PVT. LTD. (Veraval, Gujarat, India) and reacetylated to DA ranging from 10 to 55% using a procedure previously described (Vachoud et al., Carbohydr. Res. 1997, 302, 169-177). Acetic acid, oxygen peroxide (40% w/w), sulfuric acid (96% w/w), 1,2-propanediol, sodium chloride, calcium chloride, sodium nitrite and ammonium hydroxide were purchased from Sigma Aldrich. Sterile and non-pyrogenic water was purchased from Otec® . Silicon wafers (doped-P bore, orientation (100)) were purchased from Siltronix®. Glass coverslips (or substrates) (#1.5H 170$\mu$m D263 Schott glass) were purchased from IBIDI.

#### Chitosan preparation

Chitosan was subjected to filtration in order to remove insoluble and impurities prior to use. The polymer was first solubilized in an acetic acid aqueous solution, followed by successive filtrations through cellulose membrane (Millipore®) with pore sizes 3 $\mu$m, 0.8 $\mu$m, and 0.45 $\mu$m. Chitosan was then precipitated with ammonium hydroxide and washed by centrifugation with deionized water until a neutral pH was obtained (about 10 cycles). The purified chitosan was then frozen at -20°C before being finally lyophilized at - 80°C and stored at room temperature. Chitosan with various DA were prepared (Table 1) by N-reacetylation using acetic anhydride in hydro-alcoholic media, for both chitosan of different molar mass (Vachoud et al., 1997). Chitosan was first dissolved in acetic acid aqueous solution (1% w/w) overnight. A mixture of acetic anhydride and 1,2-propanediol was then added dropwise in the chitosan solution for at least 12 hours under mechanical stirring. The amount of acetic anhydride added was calculated according to the DA aimed, hypothesizing total reaction (Vachoud et al., 1997). Chitosan was then precipitated with concentrated ammonia, and finally washed and lyophilized. The DA of the different prepared chitosans was determined by NMR (Bruker Advance III, 400 MHz) using Hirai method. Das measured by $^1$H-NMR are presented in Table 1. Das obtained experimentally were in good agreement with the targeted values.

Accurate molecular mass and dispersity values were determined for all the chitosan prepared by size exclusion chromatography (SEC). For instance, starting from a chitosan having the following features: DA 1.0 $\pm$ 0.5%; $M_w$ = 156.1 kg/mol, Đ = 1.8 (MMM), a chitosan with a DA of 55% ("targeted DA") was found to have these features: $M_w$ = 192.4 kg/mol ($\pm$0.16%); Đ = 1.63.

**Table** 1. List of the different DA measured by 1 H-NMR compared to targeted values, for both medium molecular mass (Shr) chitosan and high molecular mass (Sqd) chitosan. The numerical calculation error can be up to ~2%.

| Targeted DA (%) | Measured DA (Shr) (%) | Measured DA (Sqd) (%) |
|---|---|---|
| 10 | 9 | 8.0 |
| 15 | 14.5 | 12.2 |
| 25 | 25.6 | 21.5 |
| 35 | 35.3 | 34.0 |
| 45 | 41.9 | 45.3 |
| 55 | 52.2 | 52.6 |

**Film preparation**

Silicon substrates (not the invention) or Glass substrates were cleaned from organic pollution using a piranha bath ($H_2SO_4/H_2O_2$, 7/3 v/v) heated at 150 °C for 15 minutes, and the rinsed with deionized water ($\sigma$ = 18 M$\Omega$). They were then subjected to ultra-sonication for 15 minutes and dried under a flux of clean air. The substrates were then placed into a plasma cleaner (Harrick Plasma®) for 15 minutes in order to remove inorganic traces and to generate the silanol groups at the surface for a better adsorption of chitosan polymer chains. In the meantime, the chitosan was solubilized overnight in a solution of either acetic acid or hydrochloric acid in deionized water (Otec®), under magnetic stirring at room temperature. The amount of added acid was calculated in stoichiometry compared to amine groups of chitosan in order to avoid acid hydrolysis of the glycosidic linkages. Thus, chitosan solutions with different DA and with different concentrations ranging from 0.3% to 2% were investigated in this study. The films were finally formed onto silicon or glass substrate by spin-coating at 2000 rpm until water evaporates completely. The spin-coating duration was set at 300 seconds. After spin-coating, films were stored 24 hours at room temperature without any control on the relative humidity.

**Characterization methods**

*Thickness measurement.* Spectroscopic ellipsometry measurements were carried out using an ellipsometer (SOPRA GES-5E) at an incident angle of 70°. At least three measurements were done on each film at different positions in order to verify the film homogeneity. Data were then processed using WINELLI (Sopra-SA) software. A Cauchy model was used to fit experimental data (cos $\Delta$ and tan $\Psi$, proportional to the phase and amplitude shift after reflection, respectively), in the incident spectral range of 2.0-4.5eV, depending on fits and regression qualities, to evaluate the thickness. The UV parameters A and B were respectively set to 1.53 and 0.002. Thicknesses were also measured on scratches realized on the chitosan films by tweezer, using a mechanical profilometer Veeco Instruments equipped with a cantilever of 2.5 $\mu$m in diameter. Analysis were performed with the software VISION V4.10 from Veeco Instruments.

*Surface topography.* The surface morphologies were carried out by atomic force microscopy (AFM) (CSI Nano-observer). AFM probes with spring rate ranging from 40 N/m were purchased from Bruker. The AFM images were processed using Gwyddion software.

*Contact angles measurements.* Contact angles measurements were performed using a tensiometer (Easydrop, Kruss) kit out with a camera connected to a computer equipped with drop shape analysis software. To put down the water drop on the surface, a Hamilton syringe of 1 ml and a needle of 0.5 mm diameter were used. Static measurements correspond to the angle determined 10 seconds after water drop deposition. The probe liquid used for the contact angles measurements to determine the wettability was deionized water. During stability studies films were stored at room temperature and the water contact angle was measured as a function of storage time.

**Note:** For some physicochemical characterization such as ellipsometry or FTIR analysis, the use of a reflective substrate underneath the chitosan film is required. Silicon wafers were used for this purpose. Water contact angle measurements revealed no significant differences between glass and silicon substrates, with a good homogeneity. Also, measurements of the thickness were carried by ellipsometry on silicon substrates only and by profilometry for silicon and glass substrates. Results pointed out that that the thickness of the chitosan layer spin-coated onto both types of substrates were similar. A reliable comparison can be made between results obtained for these substrates (Table 2).

**Table 2.** Contact angles obtained with deionized water as probing liquid (t = 24h), on silicon wafer or glass coverslips spin-coated with a chitosan solution of DA 55% at a concentration of 0.67% w/v. n represents the number of measurements realized on a same sample in order to obtain the uncertainty. Thickness measured by ellipsometry and profilometry on silicon wafer or glass coverslips spin-coated with a chitosan solution of DA 55% at a concentration of 0.67% w/v. n represents the number of measurements realized on a same sample in order to obtain the uncertainty.

| | Contact angle (°) | Thickness (nm) Profilometry | Thickness (nm) Ellipsometry |
|---|---|---|---|
| **Silicon wafer** | 31.7 ± 0.1 (n=3) | 33.2 ± 0.1 (n=3) | 33.9 ± 4.9 (n=3) |
| **Glass coverslip** | 28.1 ± 0.9 (n=5) | 27.6 ± 1.6 (n=5) | |

**Results**

***Influence of chitosan characteristics on film thickness.*** The thickness of chitosan thin films prepared from chitosan solutions with various concentrations, DA and molecular mass has been investigated in details. The effect of these parameters is shown in Figures 1A-B. An increase of the thickness was observed with an increase of the polymer concentration.

It appears that, for a given concentration, an increase of the DA slightly increases the thickness. For a given concentration, the thickness obtained with Sqd chitosan are higher than those obtained with Shr chitosan, as illustrated in Figures 1A-B.

***Stability of chitosan ultrathin films***

*Effect of long term and temperature storage*. Long term storage of chitosan films have been investigated at different temperatures: 4, 23 and 60 °C. Results are shown in Figure 2.

It can be observed that a saturation value for the contact angle around 65° was reached whatever the temperature of storage. However, low temperature seemed to slow down this process of contact angle increase. In this study, it seems that a delay of about 10 days is necessary in order to obtain stable chitosan surfaces. Usually, a delay of at least 5 days is respected before any uses of surfaces for micro-bacterial analysis. Biological analysis revealed no difference between films prepared after more than 5 days.

**2 - Chitosan-coated surfaces: applications**

**2.1. Material and methods**

***Chitosan coated-coverslips.*** Chitosan coated-coverslips were stored at 25°C, under a dry atmosphere.

***Channel preparation.*** The 6 channels system (sticky-Slide VI 0.4, sold by the company IBIDI) was glued immediately atop the surface of chitosan coated slide. The chitosan was rehydrated with osmosed water during at least 5 min.

***Cell adhesion and proliferation.*** A cell suspension in exponential growth (OD600nm = 0.5 ± 0.1) was diluted to obtain an OD600 nm around 0.01. Cells were diluted in Luria-Bertani (LB) medium, with a controlled ionic composition . Water was removed from the channel and the channel was rinsed 3 times with the bacterial suspension. The system was then centrifuged 3 min at 1000 rcf (Eppendorf centrifuge 5430R).

***Cell observation.*** A phase contrast microscope with a 100x objective lens was used for observations. Unless otherwise mentioned, observations were done at 25°C.

***Media preparation.*** LB (Luria-Bertani) media used for cell adhesion was prepared using 10 g/L bacto-casitone (BD, 225930), 5 g/L NaCl (Biosciences, RC-093), 5 g/L bacto Yeast extract (BD, 212750) and osmosed water supplemented with $MgCl_2$, $CaCl_2$, $ZnCl_2$ and KCl to obtain a final concentration of 0.46 μg/L Mg, 6. 15μg/L K, 3.21 μg/L Ca and 5.02 μg/L Zn.

***Batch and adhesion strength experiments.*** After centrifugation step, wells were connected to a syringe. This syringe was placed in a syringe pump (Aladdin syringe Pump WPI). For batch culture, non-adherent cells were removed through a rinse step: 1.5 ml with a 1.5ml/min flow followed by 1.5 ml with 5ml/min flow. Work flow was set at 3ml/h.

Adhesion was assessed by using an increasing flow in the channel. The shear stress was calculated by the following formula:

$$\tau = \eta \cdot 176.1 \cdot \Phi$$

wherein $\tau$ is the shear stress (dyn/cm$^2$), $\eta$ the dynamical viscosity (dyn.s/cm$^2$) and $\Phi$ the flow rate (ml/min). In absence of data about LB media dynamical viscosity, this one was assumed to be close to cell culture medium, i.e. around 0.0072 dyn.s/cm$^2$.

***CMI determination.*** Different channels were prepared simultaneously with the same cell suspension. Antibiotic was prepared at different concentrations (one channel contained only LB as a control) and added to channel just before image acquisition: each channel contain a different antibiotic concentration. A picture was taken every 2 or 3 minutes in each channel. At the end of the acquisition we determined if concentrations tested induced cell death or not.

***Antibiotic susceptibility from blood culture.*** UTI 687 was cultivated in LB medium and then diluted to obtain a cell concentration close to the one find in blood during septicemia (final concentration: 5 CFU/ml). The suspension was then inoculated in culture vial for blood culture. After over-night culture (agitation, 37°C), culture suspension was diluted in LB media for a final OD = 0.01. Cells are then prepared as describe in material and method to determine cell susceptibility to ETP.

**2.2. Results**

**Observation system**

Chitosan was coated on coverslips and a 6 channels system was stuck on top of the coverslips coated by chitosan (Figure 3A). Cells stick to chitosan after 30 min of sedimentation, but this step can be shortcut by a 3 minutes centrifugation at 1000 rcf (Figure 3B). Later experiment suggested that 150 rcf were sufficient for cell adhesion.

The 6 channels system allows a culture in batch. The channel can be connected to a syringe, automatically pushed by a push-syringe. This perfusion system is not essential for short time culture but is necessary to maintain cells in exponential growth.

**Surface screening and quality controls**

***Screening of chitosan surfaces.*** A first screening of chitosan surfaces was performed with *E. coli K12* strain. As shown in Table 3, all the chitosan tested allowed cell adhesion but low degrees of acetylation of chitosan led to abnormal cell morphologies characteristic of a physiological stress (anomalies, Figure 4A).

However, cell anomalies were not observed with the Shr chitosan coating having a degree of acetylation of 55% and prepared from solutions at concentration of 0.67% ($\chi$1) or 1.3% ($\chi$2).

Robust adhesion of cells was observed, allowing to follow cell divisions over multiple generations (Figure 4B).

***Negative control.*** Cells adhesion was also assessed on a non-coated glass coverslip. As showed in Figure 4C, even a high OD (40 fold more than with chitosan) does not allow cell adhesion. Moreover, the few adherent cells form filaments or are weakly attached, thus following divisions with time is not possible (detachment of cells).

***Sterility of the system.*** Sterility of the system was assessed by comparing two channels of chitosan. They were prepared as described above, one was filled with an *E. coli* K12 suspension and the other one with sterile LB solution. The well filled with a bacterial suspension displayed bacteria adherent to the surface while the other well did not contain any bacteria. This demonstrates the sterility of the system and confirms that the cells observed come from the *E. coli* solution.

**Table 3.** Biological response of E. coli K12 to different chitosan-coated surfaces

| Chitosan properties | | | | Biological response | | |
|---|---|---|---|---|---|---|
| Source | DA | Concentration/Thickness | Additional treatment | T0 adhesion | Proliferation | Normal morphology |
| glass | | | | - | | |
| Shr | 1%* | 0.67% | | + | + | - |
| Shr | 55% | 0.67% | | + | + | + |
| Shr | 55% | 1.3% | | + | + | + |
| Sqd | 3%* | 0.67% | | + | + | - |
| Shr | 55% | 0.67% | neutralization | + | + | + |
| Shr | 15%* | 0.67% | neutralization | + | + | - |
| Shr | 10%* | 0.67% | neutralization | + | + | - |
| Shr | 1%* | thickness: 61nm | | + | + | - |

(continued)

| Chitosan properties | | | | Biological response | | |
|---|---|---|---|---|---|---|
| Source | DA | Concentration/Thickness | Additional treatment | T0 adhesion | Proliferation | Normal morphology |
| Sqd | 3%* | thickness: 136nm | | + | + | - |
| Sqd | 3%* | thickness: 52nm | | + | + | - |
| * *not the invention* | | | | | | |

***Storage of Coated Coverslips.*** Coverslips coated with chitosan χ1 have been stored 6 months at 4°C or 6 months at 32°C. Adhesion and proliferation of *E. coli* K12 was then tested on these surfaces. No alteration was noticed with chitosan stored at 4°C but some variability of the results were observed for a chitosan stored at 32°C. Thus, it suggests that chitosan coverslips should be stored in a dry room under 30°C.

***Comparison with PLL.*** PLL (Poly-L-Lysine) has been prepared similarly to chitosan and adhesion of K12 was tested. Cells are able to adhere to PLL coated-surface but a strong filamentous phenotype is observed (Figure 4D). This support the fact that chitosan χ1 surface does promote adherence but does not suppress bacterial division for *E. coli* cells compared to PLL.

**Adhesion characterization**

***Spatial distribution of adhesion.*** Cell adhesion on chitosan χ1 and chitosan Sqd 3% 0.67% surfaces were compared by Reflection Interference Contrast Microscopy (RICM). With this technique, the closer the cells are to the surface, the darker is the contact area. A white signal shows a weak adhesion and the absence of adhesion is characterized by a background color or the alternation of white and black strips. At the beginning of the acquisition, cell bodies are black under both conditions, showing a strong adhesion of the cells to chitosan (Figure 5A and 5B). After 24 min, cells are still tightly and homogeneously attached to chitosan χ1 (Figure 5A). In contrast, on chitosan Sqd 3% 0.67%, surface adhesion is delocalized to the cell poles, leading to a loss of adhesion for the rest of the cell and apparition of cell torsion and detachment (Figure 5B). The DA of chitosan has a large impact on the cell behavior at the surface.

***Adhesion strength.*** Adhesion strength of K12 *E. coli* strain was assessed by using an increasing flow in the channel. As show in Table 4, cells adhesion is maintained under conditions used for batch culture but can also resist to strong shear stress (> 12 dyn/cm$^2$) showing a strong cellular adhesion to chitosan surface χ1.

**Table 4.** Cell adhesion according to shear stress

| Conditions | Flow | Shear stress | Adhesion |
|---|---|---|---|
| Rinse 1 | 1.5 ml/min | 1.8 dyn/cm$^2$ | + |
| Rinse 2 | 5.0 ml/min | 6.2 dyn/cm$^2$ | + |
| Acquisition | 3.0 ml/h | 0.06 dyn/cm$^2$ | + |
| Maximum flow | > 10 ml/min | > 12.3 dyn/cm$^2$ | + |

**Impact of cell-chitosan contact**

***Generation time.*** The generation time of K12 *E. coli* was estimated on the chitosan χ1 surface at 25°C. The estimated generation time was about 39 minutes, which suggests that chitosan χ1 does not impact division speed (Figure 5C).

***Effect of long time exposure to Chitosan.*** Following the protocol presented above, K12 *E. coli* cells were observed on chitosan χ1 surface. They were then incubated for 72h on the surface, without renewing growth media. After 72h, LB media was removed and fresh LB media was added. Many cells remained adherent to the surface and addition of fresh media allowed these cells to grow, without aberrant cell morphology. These data suggest that long time exposure to chitosan χ1 does not affect cell viability and cell morphology (Figure 5D).

***Adhesion according to cell cycle.*** The relation between adhesion and cell cycle was tested by using a sub-culture of *E. coli* K12 at different OD (0.05, 0.07, 0.09, 0.12, 0.23, 1.01, 1.96, 2.97, 4.55, 6.03 and 7.12), representing different time of culture and thus, different stages of the cell cycle. For each OD tested, cells adhered to chitosan $\chi$1 surface and were able to divide. Thus, cells will divide no matter at which cycle stage they are harvested and deposited on chitosan $\chi$1.

***pH effect on chitosan properties.*** It is known that chitosan displays higher antibacterial activity in an acid environment. As LB media at pH=7 was used, the effect of chitosan $\chi$1 on bacteria was investigated using a lower media pH. LB media pH was brought to 5 and adhesion and proliferation of K12 *E. coli* cells were observed. The cell behavior was similar to the one cells diluted in a pH=7 medium. Therefore, chitosan $\chi$1 coating on glass does not exhibit cytotoxic effect, even at lower pH.

**Screening for chitosan-coated surface**

***E. coli clinical strains.*** Adhesion of other laboratory and clinical *E. coli* strains to chitosan $\chi$1 surface was also studied. Strains and their properties are reported in Table 5A. The clinical strains tested were able to adhere and divide without phenotypical anomalies to chitosan $\chi$1 surface.

The adhesion of K12 and the wild type UTI 698 were assessed on chitosan Shr 55% 1.3% ($\chi$2) and the results were comparable to those obtained with chitosan $\chi$1.

***Klebsiella pneumoniae strains.*** Adhesion of *Klebsiella pneumoniae* (ATCC 700603) on chitosan $\chi$2 was tested and the strain adhered to this surface. A first screening was performed on different chitosan surfaces (Table 5B). Adhesion and cell division were also observed with chitosan Sqd 25% 0.67% and Sqd 35% 0.67%. The concentration of the chitosan solution was modified to assess the influence of the thickness of the chitosan film on cell adhesion. Most of the conditions tested with Shr chitosan (concentration 1.3%) enabled cell adhesion (Figure 6A).

The modification of Sqd chitosan concentration did not seem to affect cells adhesion and division (Table 5B).

*K. pneumoniae* LM21 strain was tested with Shr 35% 0.67% chitosan surface. On this surface, the adhesion is optimal, as well as divisions (Figure 6B). LM21 strain was also tested with 5 other chitosan surfaces and biological responses obtained with this strain were similar to those obtained with ATCC 700603. (Table 5B).

**Table 5. (A)** Screening of E. coli strain. **(B)** Screening of chitosan surfaces for Klebsiella pneumoniae ATCC 700603 and LM21 (a positive biological response refers to a good adhesion and proliferation).

**A**

| Strain | Particularity | Adhesion |
|---|---|---|
| K12 | | + |
| MG1655 | | + |
| 227 | | + |
| 219 | | + |
| 53 | | + |
| 106 | | + |
| 704 | | + |
| 678 | | + |
| 677 | | + |
| 668 | | + |
| 773 | ESBL | + |
| 687 | digestive/ESBL | + |
| 698 | WT | + |

**B**

| Chitosan properties | | | Biological response | |
|---|---|---|---|---|
| Source | DA | concentration (%) | KP700603 | LM21 |
| Shr | 1%* | 0.67 | - | - |
| Shr | 10%* | 0.67 | - | - |
| Sqd | 10%* | 0.67 | - | |
| Sqd | 25% | 0.67 | +++ | |
| Sqd | 35% | 0.67 | +++ | +++ |
| Sqd | 45% | 0.67 | ++ | |
| Sqd | 55% | 0.67 | ++ | |
| Shr | 35% | 1.3 | +++ | |

(continued)

**B**

| | Chitosan properties | | Biological response | |
|---|---|---|---|---|
| Source | DA | concentration (%) | KP700603 | LM21 |
| Shr | 45% | 1.3 | + | |
| Shr | 55% | 1.3 | +++ | +++ |
| Sqd | 1%* | 0.4 | | - |
| Sqd | 25% | 0.4 | | +++ |
| Sqd | 35% | 0.4 | | + |
| Sqd | 45% | 0.4 | | +++ |

**not the invention*

**Medium composition**

***Determination of an optimal medium composition.*** The media composition could be a limiting factor for adhesion: modification of ionic composition may lead to a modification of the adhesion response. It appeared that 0.46 μg/L of Mg, 6.15μg/L of K, 2.31 μg/L of Ca and 5.02 μg/L of Zn enabled an optimal adhesion. This analysis only proposes an optimal controlled medium composition for bacterial adhesion and proliferation on chitosan χ1 based on *E. coli* K12 observations, and intends in no way to limit methods described herein to this medium composition.

**Antibiotics susceptibility**

***Visualization of antibiotics susceptibility.*** Two channels were prepared with K12, identically. Before starting picture acquisition, one channel was rinsed with LB media, the other one with LB media supplemented with 100μg/ml of ampicillin. In the presence of ampicillin, cells extended but did not divide (no septum formation), leading to the disruption of plasma membrane and cell death (Figure 7A). Morphological changes were observed earlier than 1 hour and cell death imaged by lysis occurred right after 2 hours of incubation.

***Dose-dependent answer.*** Ertapenem (ETP) is an antibiotic used in the medical field in order to determine MIC. ETP was tested at different concentrations on UTI 227 and for each cell observed, the time for cell death was quantified (Figures 7B and 7C). The effect of ETP was significantly faster for a higher concentration. Similarly, UTI 687 was contacted with different concentration of mecillinam (MEC), and the cell width was quantified. An augmentation was observed at 2 mg/L of MEC after 1 hour of contact (Figure 7D). These results suggest that the system is optimal to visualize antibiotic dose-dependent effect on different cells strains.

***Minimum inhibitory concentration.*** Several experiments were performed with different ETP concentrations added to UTI 227 cells. It was observed that ETP had a cytotoxic effect above 0.01 mg/L, thus MIC of ETP is lower or equal to 0.05 for UTI 227 (Figure 7E). According to Vitek® results, MIC is less or equal to 0.5 therefore the MIC determined here is concordant and even more precise than the results obtained with Vitek® (Figure 8).

***Growth curves and estimation of the minimal diagnostic time.*** The effects of Ertapenem on growth of *E. coli* clinical strains was studied on a chitosan χ1 surface (Figure 9). Growth curves were measured for strain UTI227 with varying concentrations of ETP.

An estimation of the growth rate for varying time spans for all assays was performed and the fraction of assays for which the response could be ascertained with a 95% confidence interval, was determined for each time span (Figure 10). The minimal diagnostic time can thereby be estimated.

All experiments for MIC determination were performed with chitosan χ1 and are shown in Table 6. A comparison between MIC determined with the chitosan system and Vitek is also shown in this Table. For each conditions, a similar result was obtained.

**Table 6.** MIC determination for different antibiotics. (Each line is a different experiment. Concentrations in mg/L. "Growth" : cell growth. "Death" : bactericide/bacteriostatic effect of the antibiotic).

| Clinical Strain | Experiment | Antibiotic | Concentration (mg/ml) | CMI (Vitek) | Outcome | CMI (measured) |
|---|---|---|---|---|---|---|
| UTI227 | 1 | Ertapenem | **0** | ≤ 0.5 | Growth | ≤ 1 |
| | | | **0** | | Growth | |
| | | | **1** | | Growth | |
| | | | **1** | | Death | |
| | 2 | | **0** | | Growth | ≤ 0.25 |
| | | | **0.25** | | Death | |
| | | | **0.5** | | Death | |
| | | | **0.75** | | Death | |
| | 3 | | **0** | | Growth | ≤ 0.1 |
| | | | **0.1** | | Death | |
| | | | **0.25** | | Death | |
| | | | **0.5** | | Death | |
| | | | **0.75** | | Death | |
| | 4 | | **0** | | Growth | ≤ 0.05 |
| | | | **0.05** | | Death | |
| | | | **0.125** | | Death | |
| | | | **0.25** | | Death | |
| | 5 | | **0** | | Growth | **0.01≤ CMI≤ 0.05** |
| | | | **0.01** | | Growth | |
| | | | **0.05** | | Death | |
| | | | **0.25** | | Death | |
| | | | **0.5** | | Death | |
| | 1 | Mecillinam | **0** | 8 | Growth | ≤ 8 |
| | | | **4** | | Growth | |
| | | | **8** | | Death | |

| | | | | | | |
|---|---|---|---|---|---|---|
| | | | **12** | | Death | |
| | 2 | | **0** | | Growth | ≤ 8 |
| | | | **4** | | Growth | |
| | | | **8** | | Death | |
| | | | **12** | | Death | |
| | | | **16** | | Death | |
| UTI704 | 1 | Mecillinam | **0** | 2 | Growth | ≤ 2 |
| | | | **0.25** | | Growth | |
| | | | **0.5** | | Growth | |
| | | | **1** | | Growth | |
| | | | **2** | | Death | |
| UTI687 | 1 | Ofloxacin | **0** | 0.5 | Growth | ≤ 0.25 |
| | | | **0.25** | | Death | |
| | | | **0.5** | | Death | |
| | | | **1** | | Death | |
| | | | **2** | | Death | |
| UTI698 | 1 | Mecillinam | **0** | <1 | Growth | ≤ 1 |
| | | | **1** | | Death | |
| | | | **2** | | Death | |
| | | | **2** | | Death | |
| | | | **5** | | Death | |

***Antibiotic susceptibility from blood culture.*** Cells grown in blood culture vial were able to adhere and proliferate on the chitosan $\chi 1$ surface, but a lot of debris were present. These debris probably originate from blood culture media. However, it was possible to determine a CMI: $CMI_{\chi 1} \leq 1$, which is coherent with the expected values. This experiment suggests that it would be possible to by-pass the culture step after blood culture for CMI determination, even though blood culture vial also contains blood from the patient, in standard conditions, which could interfere with chitosan.

**Improvement of cell death detection**

Propidium iodide (PI) is used as a DNA stain which cannot cross the membrane of live cells, making it useful to differentiate dead, dying and healthy cells. K12 cells were immobilized to commercial chitosan on microfluidic chamber (channels in PDMS) in presence or absence of EtOH 70%. Channels were rinsed with LB, 50μL/mL PI. After 30 seconds, all the cells incubated with EtOH were fluorescent, and control cells remained non-fluorescent (Figure 11A). Thus, it is possible to use fluorescent molecule in combination with chitosan. In that case, it is possible to discriminate healthy and dying cells using PI in less than 30 seconds of dye incubation.

Following the protocol described in material and methods, UTI 687 cells were contacted with chitosan $\chi 1$ surface. Before acquisition, channel was rinsed with LB supplemented with 3 mg/L of ETP and 50μL/mL of PI (Figure 11B). During acquisition, some cells turned in red, in correlation with a dead/dying phenotype observed. This demonstrates that PI can be used to easily identify antibiotic effect on cells.

## Claims

**1.** A device for microorganism growth comprising a glass substrate and a chitosan film deposited thereon, wherein said chitosan has the following features:

- a molar mass between 100,000 and 900,000 g/mol, preferably between 150,000 and 300,000 g/mol;
- a degree of acetylation between 25 and 65 %, preferably between 32 and 57 %,

wherein the degree of acetylation is the percentage of the number of acetylated units over the total number of units constituting the chitosan polymer,

where the acetylated units are N-acetylglucosamine units and the units constituting the chitosan polymer are N-acetylglucosamine and glucosamine units.

**2.** The device according to claim 1, wherein the thickness of said chitosan film is comprised between 5 and 500 nm, preferably between 40 and 250 nm.

**3.** The device according to claim 1 or 2, wherein the surface area of said chitosan film is comprised between 10 $\mu m^2$ and 80 $cm^2$.

**4.** The device according to any of claims 1 to 3, further comprising a microchannels system set onto said chitosan film.

**5.** A use of the device according to any of claims 1 to 4, for *in vitro* detecting and/or observing, microorganisms and/or impacts thereon due to microenvironment changes.

**6.** A use of the device according to any of claims 1 to 4, for *in vitro* determining the resistance or susceptibility of microorganisms to bioactive agents, said microorganisms being preferably bacteria.

**7.** A use of the device according to any of claims 1 to 4, for *in vitro* screening for an antimicrobial agent of at least one microorganism.

**8.** A use of the device according to any of claims 1 to 4, for *in vitro* diagnosing a microbial infection in a patient.

**9.** A method for *in vitro* determining the resistance or susceptibility of microorganisms to bioactive agents, comprising the steps of:

(a) contacting a sample containing microorganisms with the chitosan film of the device as defined in anyone of claims 1-4;
(b) maintaining said microorganisms in contact with the film for a first period of time;
(c) adding a solution comprising at least one bioactive agent;
(d) maintaining said microorganisms and solution in contact with the film for a second period of time;
(e) detecting growth of individual microorganisms after the addition of said bioactive agent, more specifically by microscopy.

**10.** The use or the method according to any one of claims 5 to 9, wherein said microorganisms are bacteria, preferably enterobacteria such as Citrobacter, Enterobacter, Escherichia, Klebsiella, Morganella, Pectobacterium, Plesiomonas, Proteus, Salmonella, Shigella, Serratia or Yersinia.

**11.** The use or the method according to claim 10, wherein said bacteria come from a bacteria culture, such as a primoculture or a blood culture, a subculture thereof, and/or a body fluid, such as urine.

**12.** The use or method according to any one of claims 10 and 11, wherein said bioactive agent is an antimicrobial agent, preferably an antibiotic, and/or wherein the solution added in step (c) further comprises a DNA stain, such as propidium iodide, and/or wherein the determination of the resistance or susceptibility of microorganisms to at least one bioactive agent requires less than 180 minutes, preferably less than 90 minutes, more preferably less than 60 minutes, after adding said solution comprising at least one bioactive agent in step (c), and/or wherein the minimum inhibitory concentration is determined.

**13.** A method for preparing a device as defined in claim 2, comprising the steps of:

(a) preparing a chitosan having the following features:

- a molar mass between 100,000 and 900,000 g/mol, preferably between 150,000 and 300,000 g/mol,
- a degree of acetylation between 25 and 65 %, preferably between 32 and 57 %;

(b) preparing an acidic aqueous solution of said chitosan, which chitosan concentration is comprised between 0.1 and 3 % (w/w), preferably between 0.5 and 1.5 % (w/w);
(c) depositing the solution obtained in step (b) onto the substrate and forming a film of chitosan, preferably by spin coating, under conditions allowing to obtain a film thickness between 5 and 500 nm, preferably between 40 and 220 nm.

**14.** A chitosan material, wherein the chitosan has the following features:

- a molar mass between 100,000 and 900,000 g/mol, preferably between 120,000 and 610,000 g/mol, more preferably between 150,000 and 300,000 g/mol,
- a degree of acetylation between 32 and 57 %,

and wherein the chitosan is in the form of a film, preferably a film having a thickness between 5 and 500 nm, preferably between 40 and 220 nm,
wherein the degree of acetylation is the percentage of the number of acetylated units over the total number of units constituting the chitosan polymer,
where the acetylated units are N-acetylglucosamine units and the units constituting the chitosan polymer are N-acetylglucosamine and glucosamine units.

**15.** A use of a chitosan material according to claim 14, as an *in vitro* adhesion material of microorganisms, preferably bacteria.

## Patentansprüche

**1.** Vorrichtung zum Aufwuchs von Mikroorganismen, umfassend ein Glassubstrat und einen darauf abgeschiedenen Chitosanfilm, wobei das Chitosan die folgenden Merkmale aufweist:

- eine Molmasse zwischen 100.000 und 900.000 g/mol, vorzugsweise zwischen 150.000 und 300.000 g/mol
- einen Acetylierungsgrad zwischen 25 und 65 %, vorzugsweise zwischen 32 und 57 %, wobei der Acetylierungsgrad der Prozentsatz der Anzahl an acetylierten Einheiten gegenüber der Gesamtzahl an Einheiten ist, aus denen das Chitosan-Polymer besteht,

wobei die acetylierten Einheiten N-Acetylglucosamin-Einheiten sind und die Einheiten, aus denen das Chitosan-Polymer besteht, N-Acetylglucosamin- und Glucosamin-Einheiten sind.

**2.** Vorrichtung nach Anspruch 1, wobei die Dicke dieses Chitosanfilms zwischen 5 und 500 nm, vorzugsweise zwischen 40 und 250 nm, liegt.

**3.** Vorrichtung nach Anspruch 1 oder 2, wobei die Oberfläche dieses Chitosanfilms zwischen 10 $\mu m^2$ und 80 $cm^2$ beträgt.

**4.** Vorrichtung nach einem der Ansprüche 1 bis 3, ferner umfassend ein Mikrokanalsystem, das auf diesem Chitosanfilm angeordnet ist.

**5.** Verwendung der Vorrichtung nach einem der Ansprüche 1 bis 4 zum in-vitro-Nachweisen und/oder -Beobachten von Mikroorganismen und/oder Einwirkungen darauf aufgrund von Mikroumgebungsveränderungen.

**6.** Verwendung der Vorrichtung nach einem der Ansprüche 1 bis 4 zur *in-vitro*-Bestimmung der Resistenz oder Empfindlichkeit von Mikroorganismen gegenüber bioaktiven Mitteln, wobei die Mikroorganismen vorzugsweise Bakterien sind.

**7.** Verwendung der Vorrichtung nach einem der Ansprüche 1 bis 4 zum *in-vitro*-Screening auf einen antimikrobiellen Wirkstoff von mindestens einem Mikroorganismus.

**8.** Verwendung der Vorrichtung nach einem der Ansprüche 1 bis 4 zur *in-vitro*-Diagnose einer mikrobiellen Infektion bei einem Patienten.

**9.** Verfahren zur *in-vitro*-Bestimmung der Resistenz oder Empfindlichkeit von Mikroorganismen gegenüber bioaktiven Mitteln, umfassend die Schritte:

(a) Inkontaktbringen einer Probe, die Mikroorganismen enthält, mit dem Chitosanfilm der Vorrichtung nach einem der Ansprüche 1 bis 4;
(b) Halten der Mikroorganismen in Kontakt mit dem Film für einen ersten Zeitraum;
(c) Zugeben einer Lösung, die mindestens ein bioaktives Mittel umfasst;
(d) Halten dieser Mikroorganismen und dieser Lösung in Kontakt mit dem Film für einen zweiten Zeitraum;
(e) Nachweis des Wachstums einzelner Mikroorganismen nach Zugabe dieses bioaktiven Mittels, insbesondere durch Mikroskopie.

10. Verwendung oder Verfahren nach einem der Ansprüche 5 bis 9, wobei diese Mikroorganismen Bakterien sind, vorzugsweise Enterobakterien wie Citrobacter, Enterobacter, Escherichia, Klebsiella, Morganella, Pectobacterium, Plesiomonas, Proteus, Salmonella, Shigella, Serratia oder Yersinia .

11. Verwendung oder Verfahren nach Anspruch 10, wobei die Bakterien aus einer Bakterienkultur wie einer Primärkultur oder einer Blutkultur, einer Subkultur davon und/oder einer Körperflüssigkeit wie Urin stammen.

12. Verwendung oder Verfahren nach einem der Ansprüche 10 und 11, wobei dieses bioaktive Mittel ein antimikrobielles Mittel ist, vorzugsweise ein Antibiotikum, und/oder wobei die in Schritt (c) zugegebene Lösung ferner einen DNA-Farbstoff, wie Propidium-Iodid, umfasst, und/oder wobei die Bestimmung der Resistenz oder Empfindlichkeit von Mikroorganismen gegenüber mindestens einem bioaktiven Mittel weniger als 180 Minuten, vorzugsweise weniger als 90 Minuten, stärker bevorzugt weniger als 60 Minuten, nach Zugabe der Lösung in Schritt (c), die mindestens ein bioaktives Mittel umfasst, erfordert, und/oder wobei die minimale Hemmkonzentration bestimmt wird.

13. Verfahren zum Herstellen einer Vorrichtung nach Anspruch 2, umfassend die Schritte:

(a) Herstellen eines Chitosans mit den folgenden Merkmalen:

- einer Molmasse zwischen 100.000 und 900.000 g/mol, vorzugsweise zwischen 150.000 und 300.000 g/mol,
- einen Acetylierungsgrad zwischen 25 und 65 %, vorzugsweise zwischen 32 und 57 %;

(b) Herstellen einer sauren wässrigen Lösung dieses Chitosans, wobei die Chitosankonzentration zwischen 0,1 und 3 % (w/w), vorzugsweise zwischen 0,5 und 1,5 % (w/w) liegt;
(c) Abscheiden der in Schritt (b) erhaltenen Lösung auf dem Substrat und Bilden eines Films aus Chitosan, vorzugsweise durch Schleuderbeschichtung ("spin coating"), unter Bedingungen, die es ermöglichen, eine Filmdicke zwischen 5 und 500 nm, vorzugsweise zwischen 40 und 220 nm zu erhalten.

14. Chitosan-Material, wobei das Chitosan die folgenden Merkmale aufweist:

- eine Molmasse zwischen 100.000 und 900.000 g/mol, bevorzugt zwischen 120.000 und 610.000 g/mol, besonders bevorzugt zwischen 150.000 und 300.000 g/mol,
- einen Acetylierungsgrad zwischen 32 und 57 %,
und wobei das Chitosan in Form eines Films vorliegt, vorzugsweise eines Films mit einer Dicke zwischen 5 und 500 nm, vorzugsweise zwischen 40 und 220 nm,
wobei der Acetylierungsgrad der Prozentsatz der Anzahl an acetylierten Einheiten gegenüber der Gesamtzahl an Einheiten ist, aus denen das Chitosan-Polymer besteht,
wobei die acetylierten Einheiten N-Acetylglucosamin-Einheiten sind und die Einheiten, aus denen das Chitosan-Polymer besteht, N-Acetylglucosamin- und Glucosamin-Einheiten sind.

15. Verwendung eines Chitosanmaterials nach Anspruch 14 als *in* vitro-Adhäsionsmaterial von Mikroorganismen, vorzugsweise Bakterien.

## Revendications

1. Dispositif pour la croissance de microorganismes, comprenant un substrat en verre et un film de chitosane déposé sur celui-ci, dans lequel ledit chitosane a les caractéristiques suivantes :

- une masse molaire comprise entre 100 000 et 900 000 g/mol, de préférence entre 150 000 et 300 000 g/mol ;
- un degré d'acétylation compris entre 25 et 65 %, de préférence entre 32 et 57 %, lequel degré d'acétylation étant le pourcentage du nombre de motifs acétylés par rapport au nombre total de motifs constituant le polymère de chitosane,

dans lequel les motifs acétylés sont des motifs N-acétylglucosamine et les motifs constituant le polymère de chitosane sont des motifs N-acétylglucosamine et glucosamine.

2. Dispositif selon la revendication 1, dans lequel l'épaisseur dudit film de chitosane est comprise entre 5 et 500 nm,

de préférence entre 40 et 250 nm.

3. Dispositif selon la revendication 1 ou 2, dans lequel la superficie dudit film de chitosane est comprise entre 10 $\mu m^2$ et 80 $cm^2$.

4. Dispositif selon l'une quelconque des revendications 1 à 3, comprenant en outre un système de microcanaux établi sur ledit film de chitosane.

5. Utilisation du dispositif selon l'une quelconque des revendications 1 à 4 pour la détection et/ou l'observation *in vitro* de microorganismes et/ou d'impacts sur ceux-ci dus à des changements microenvironnementaux.

6. Utilisation du dispositif selon l'une quelconque des revendications 1 à 4 pour la détermination *in vitro* de la résistance ou de la sensibilité de microorganismes à des agents bioactifs, lesdits microorganismes étant de préférence des bactéries.

7. Utilisation du dispositif selon l'une quelconque des revendications 1 à 4 pour le criblage *in vitro* d'un agent antimicrobien contre au moins un microorganisme.

8. Utilisation du dispositif selon l'une quelconque des revendications 1 à 4 pour le diagnostic *in vitro* d'une infection microbienne chez un patient.

9. Méthode pour déterminer *in vitro* la résistance ou la sensibilité de microorganismes à des agents bioactifs, comprenant les étapes de :

   (a) mise en contact d'un échantillon contenant des microorganismes avec le film de chitosane du dispositif tel que défini dans l'une quelconque des revendications 1 à 4 ;
   (b) maintien desdits microorganismes en contact avec le film pendant une première période de temps ;
   (c) ajout d'une solution comprenant au moins un agent bioactif ;
   (d) maintien desdits microorganismes et de ladite solution en contact avec le film pendant une deuxième période de temps ;
   (e) détection de la croissance de microorganismes individuels après l'ajout dudit agent bioactif, plus spécifiquement par microscopie.

10. Utilisation ou méthode selon l'une quelconque des revendications 5 à 9, dans laquelle lesdits microorganismes sont des bactéries, de préférence des entérobactéries telles que Citrobacter, Enterobacter, Escherichia, Klebsiella, Morganella, Pectobacterium, Plesiomonas, Proteus, Salmonella, Shigella, Serratia ou Yersinia.

11. Utilisation ou méthode selon la revendication 10, dans laquelle lesdites bactéries proviennent d'une culture bactérienne, telle qu'une primoculture ou une hémoculture, d'une sous-culture de celle-ci, et/ou d'un fluide corporel, tel que l'urine.

12. Utilisation ou méthode selon l'une quelconque des revendications 10 et 11, dans laquelle ledit agent bioactif est un agent antimicrobien, de préférence un antibiotique, et/ou dans laquelle la solution ajoutée à l'étape (c) comprend en outre un colorant d'ADN, tel que l'iodure de propidium, et/ou dans laquelle la détermination de la résistance ou de la sensibilité de microorganismes à au moins un agent bioactif requiert moins de 180 minutes, de préférence moins de 90 minutes, mieux encore moins de 60 minutes, après l'ajout de ladite solution comprenant au moins un agent bioactif à l'étape (c), et/ou dans laquelle la concentration inhibitrice minimale est déterminée.

13. Méthode pour préparer un dispositif tel que défini dans la revendication 2, comprenant les étapes de :

   (a) préparation d'un chitosane ayant les caractéristiques suivantes :

      - une masse molaire comprise entre 100 000 et 900 000 g/mol, de préférence entre 150 000 et 300 000 g/mol ;
      - un degré d'acétylation compris entre 25 et 65 %, de préférence entre 32 et 57 % ;

   (b) préparation d'une solution aqueuse acide dudit chitosane, dont la concentration de chitosane est comprise entre 0,1 et 3 % (p/p), de préférence entre 0,5 et 1,5 % (p/p) ;
   (c) dépôt de la solution obtenue à l'étape (b) sur le substrat et formation d'un film de chitosane, de préférence

par revêtement centrifuge, dans des conditions permettant l'obtention d'une épaisseur de film comprise entre 5 et 500 nm, de préférence entre 40 et 220 nm.

**14.** Matériau de chitosane, dans lequel le chitosane a les caractéristiques suivantes :

- une masse molaire comprise entre 100 000 et 900 000 g/mol, de préférence entre 120 000 et 610 000 g/mol, mieux encore entre 150 000 et 300 000 g/mol,
- un degré d'acétylation compris entre 32 et 57 %,

et dans lequel le chitosane est sous la forme d'un film, de préférence d'un film ayant une épaisseur comprise entre 5 et 500 nm, de préférence entre 40 et 220 nm,
dans lequel le degré d'acétylation est le pourcentage du nombre de motifs acétylés par rapport au nombre total de motifs constituant le polymère de chitosane,
dans lequel les motifs acétylés sont des motifs N-acétylglucosamine et les motifs constituant le polymère de chitosane sont des motifs N-acétylglucosamine et glucosamine.

**15.** Utilisation d'un matériau de chitosane selon la revendication 14, en tant que matériau d'adhésion *in vitro* de microorganismes, de préférence de bactéries.

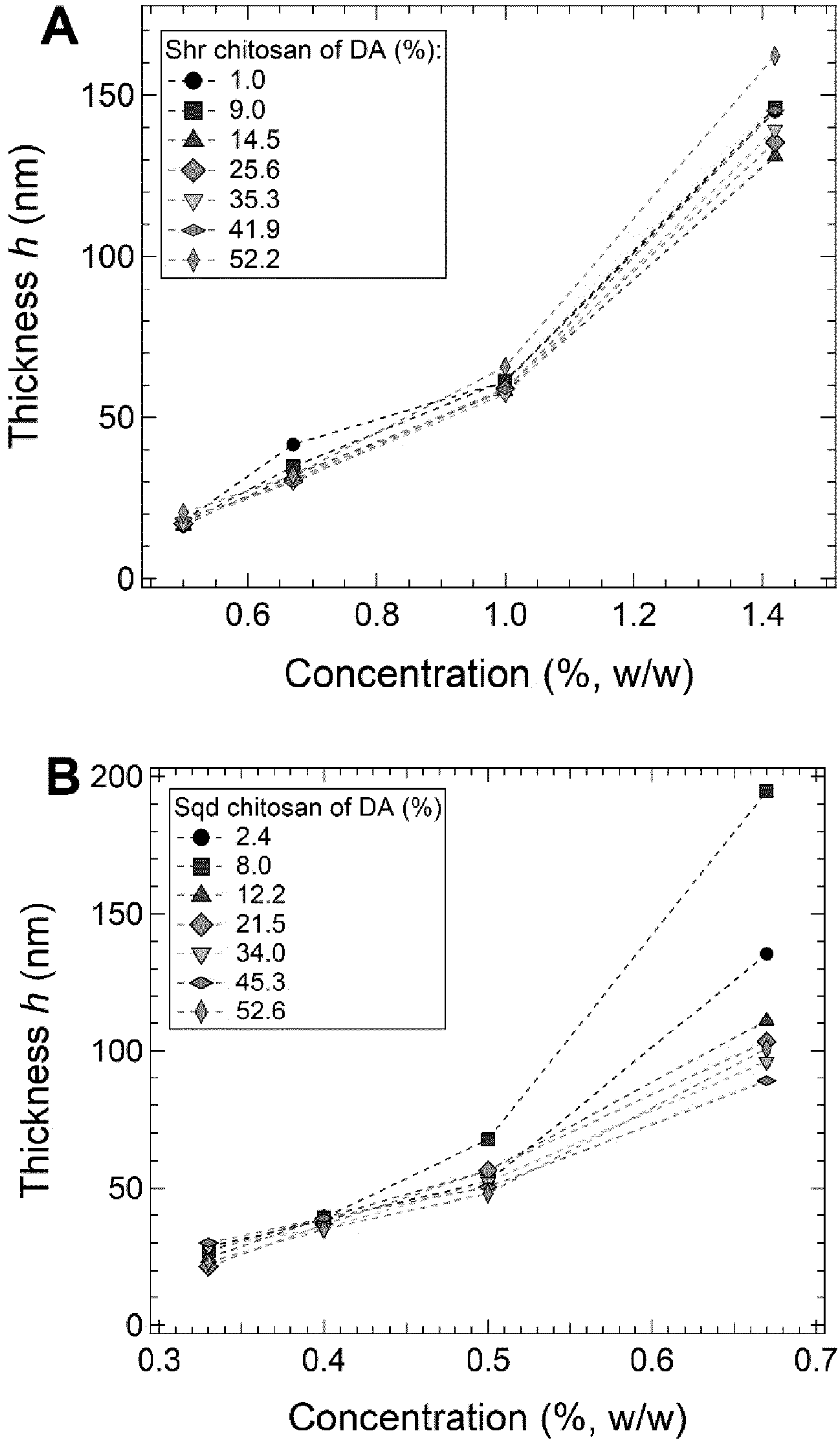
A
Shr chitosan of DA (%):
1.0
9.0
14.5
25.6
35.3
41.9
52.2
Thickness h (nm)
0
50
100
150
0.6
0.8
1.0
1.2
1.4
Concentration (%, w/w)
B
Sqd chitosan of DA (%)
2.4
8.0
12.2
21.5
34.0
45.3
52.6
Thickness h (nm)
0
50
100
150
200
0.3
0.4
0.5
0.6
0.7
Concentration (%, w/w)

**Figures 1**

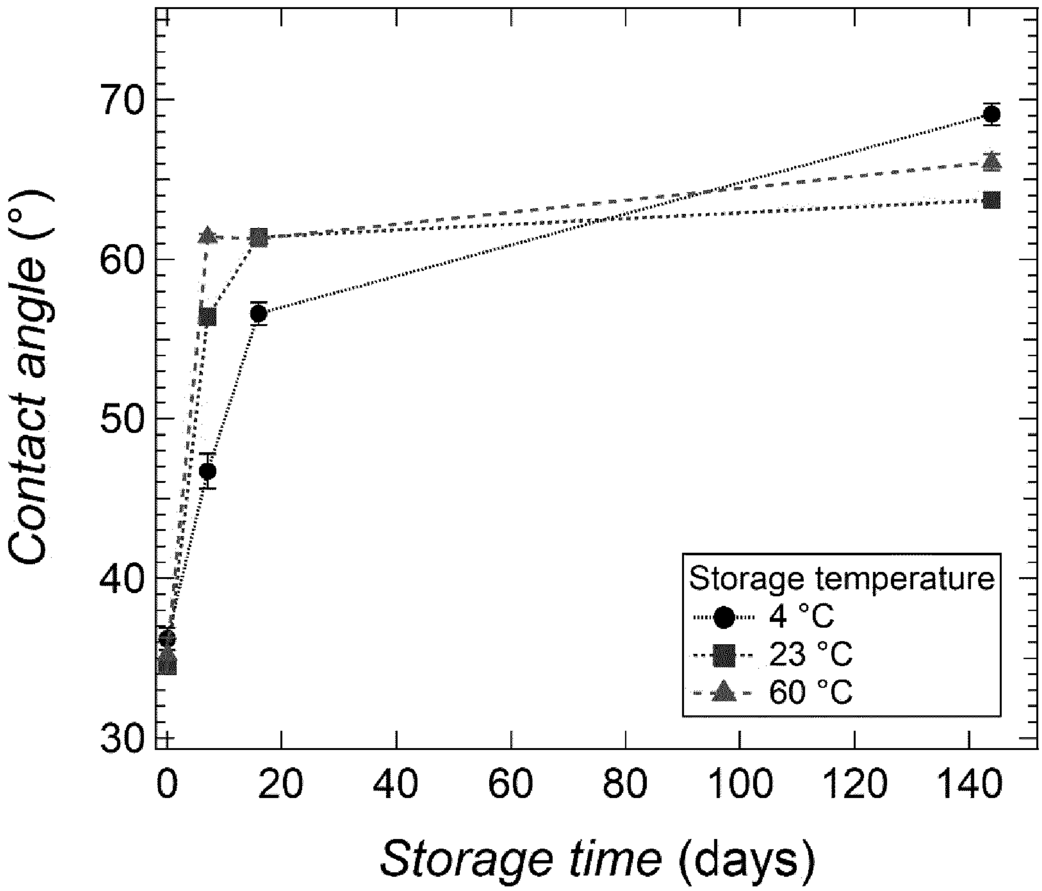


**Figure 2**

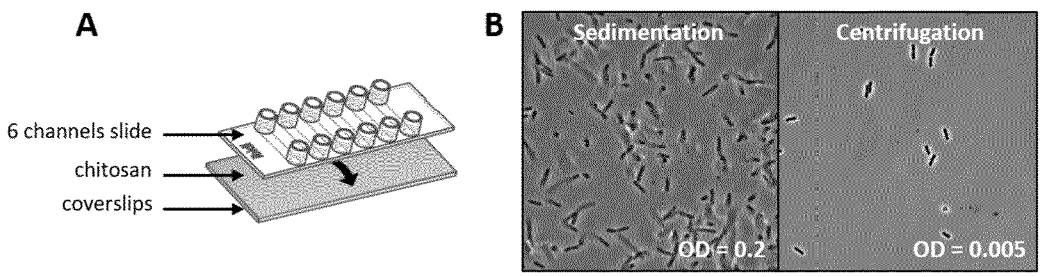


**Figures 3**

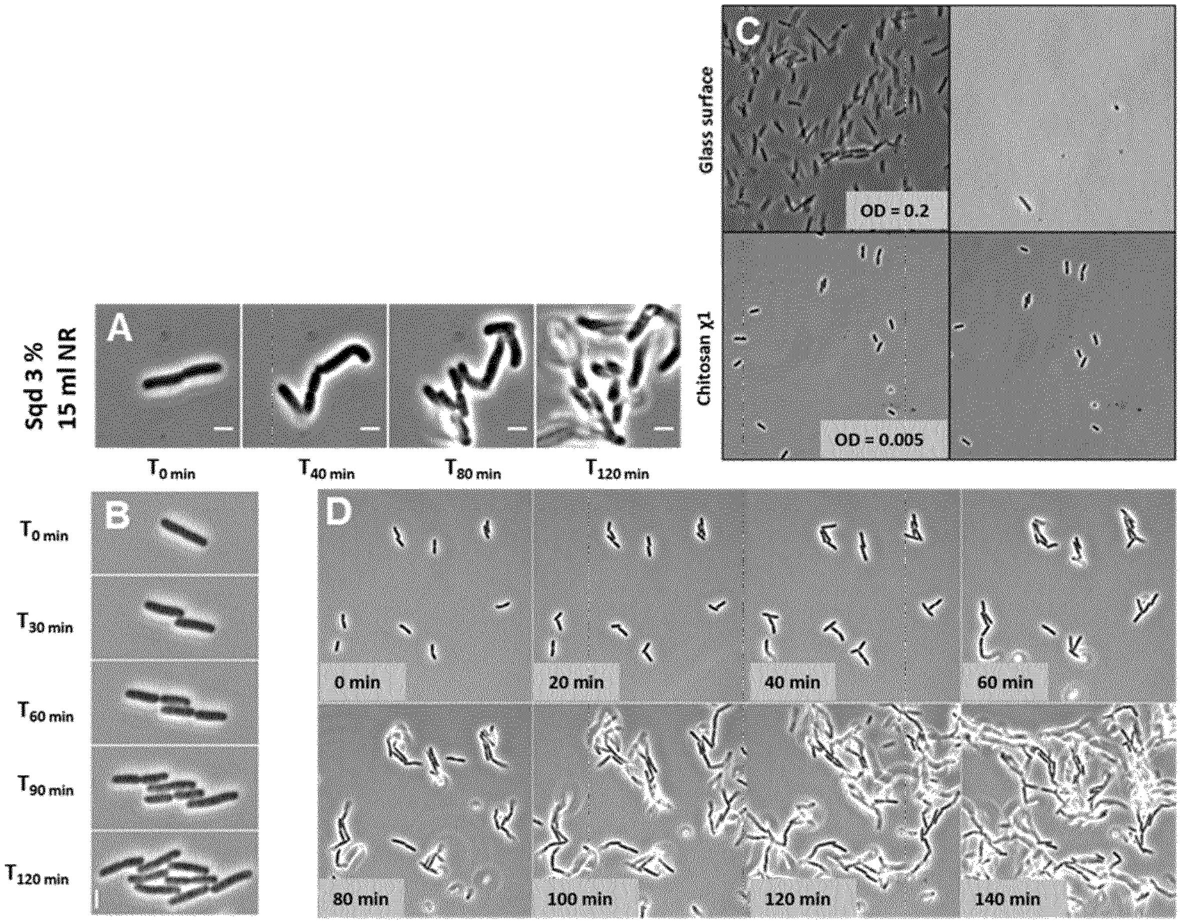


Figures 4

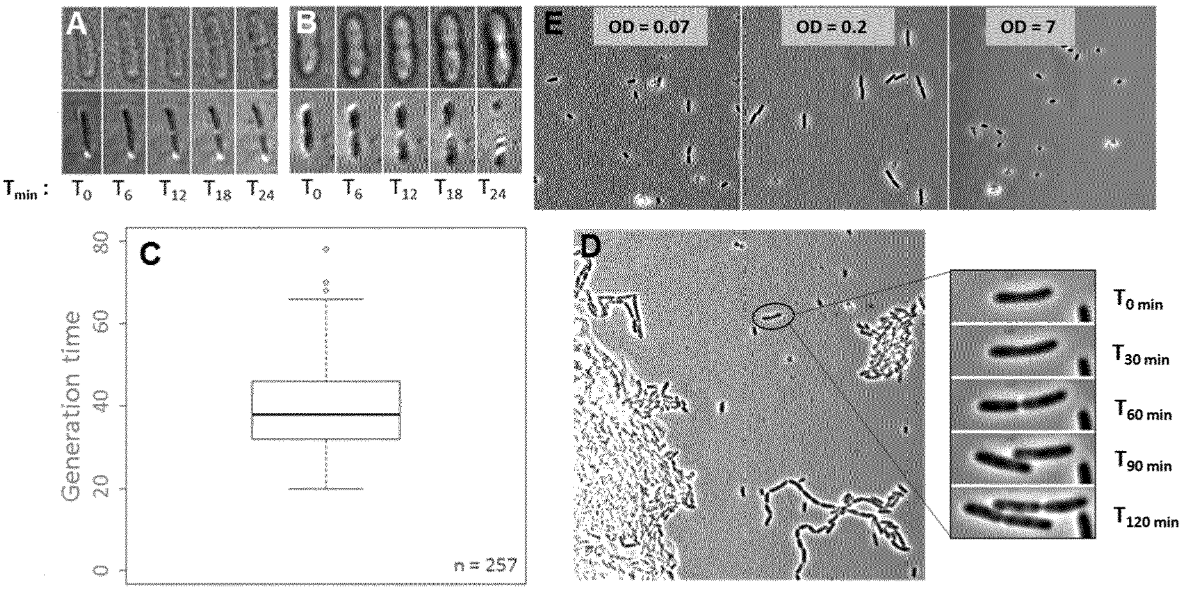


Figures 5

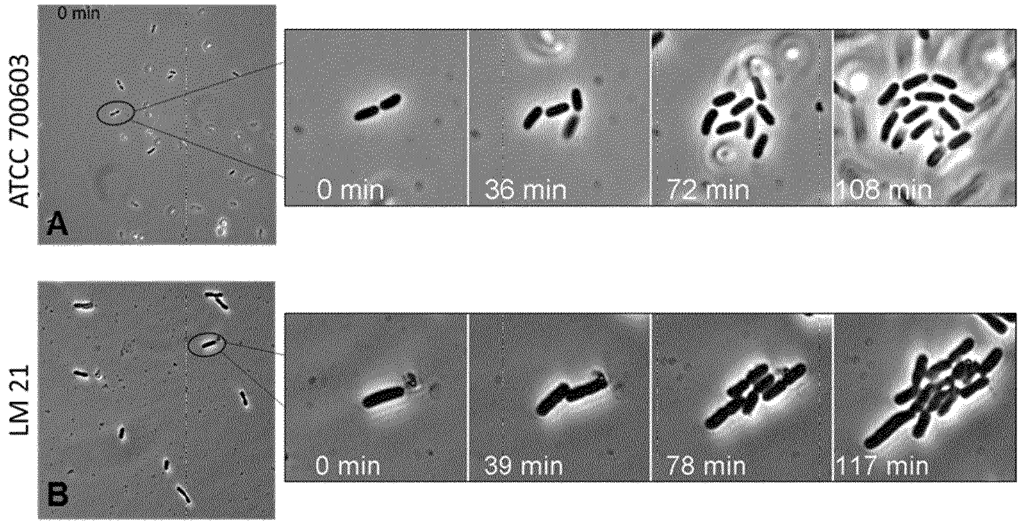


Figures 6

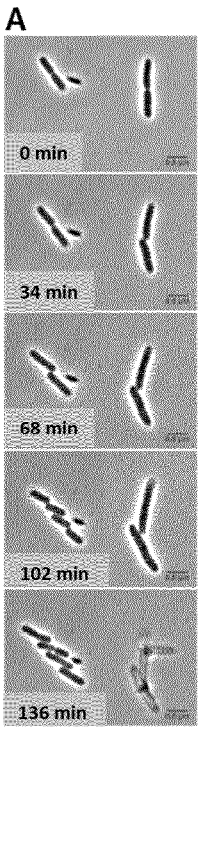


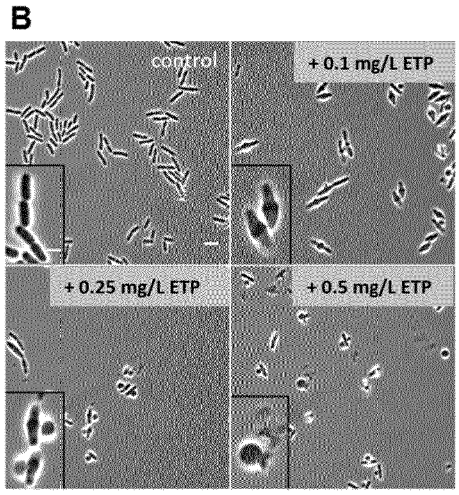


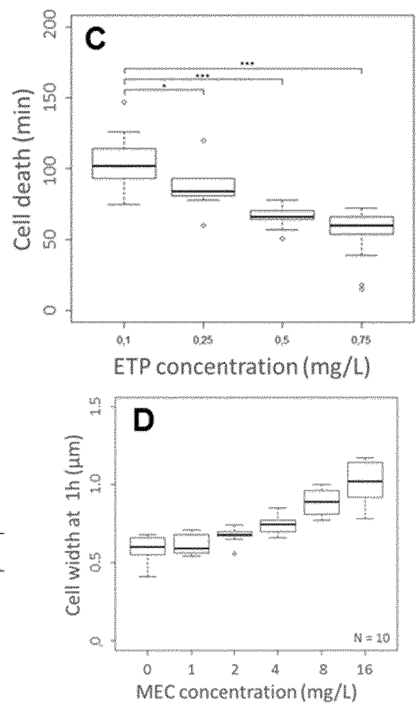


E

| ETP(mg/L) | | | | | | | | |
|---|---|---|---|---|---|---|---|---|
| 0 | 0.01 | 0.05 | 0.10 | 0.13 | 0.25 | 0.50 | 0.75 | 1 |
| + | | | | | | | | - |
| + | | | | | - | - | - | |
| + | + | - | | | - | - | | |
| + | | - | | - | - | | | |
| + | | | - | | - | - | - | |

Figures 7

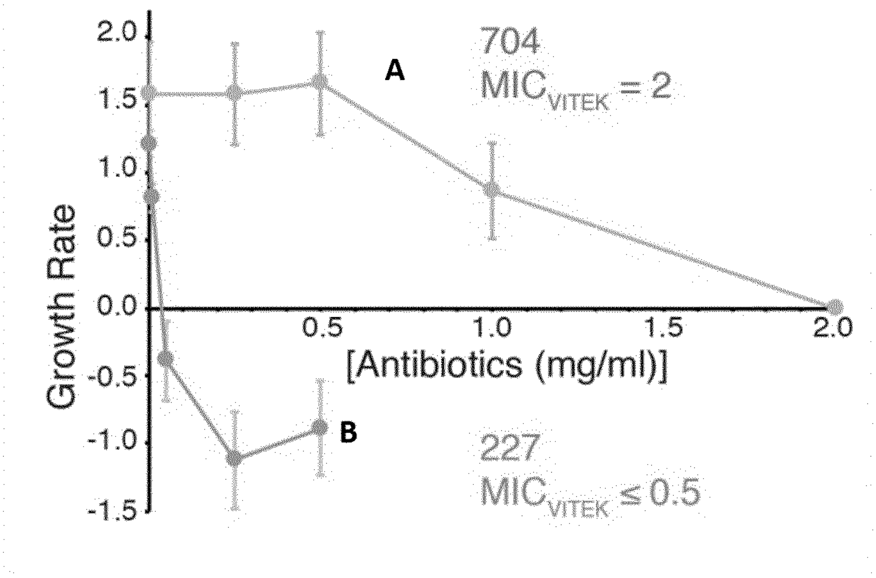


Figure 8

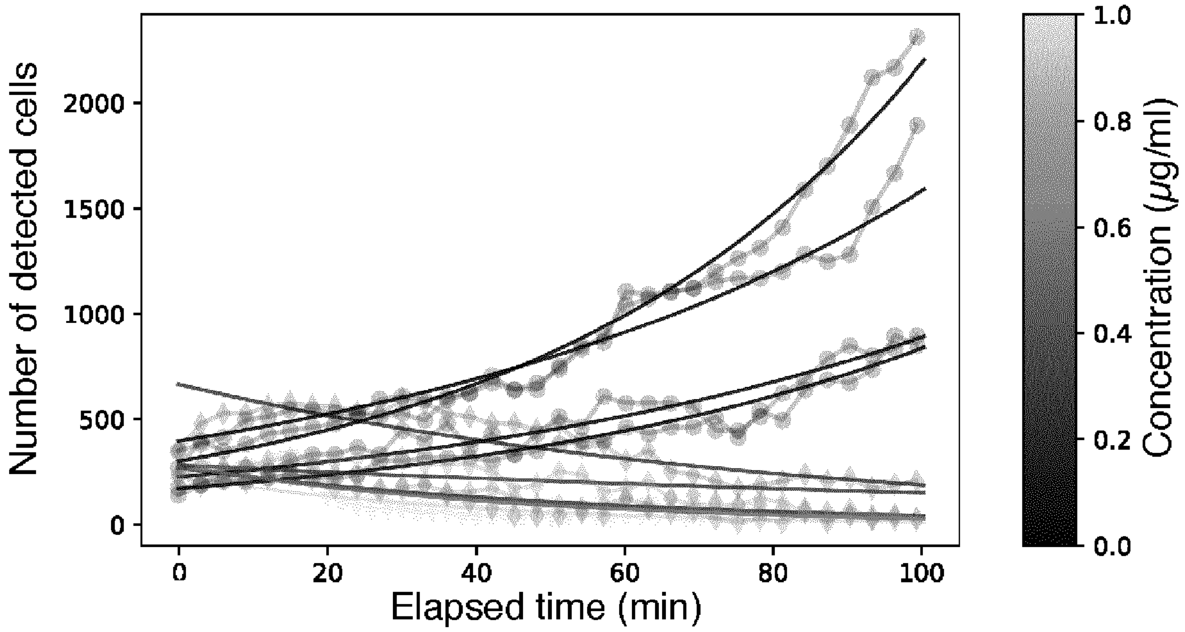


Figure 9

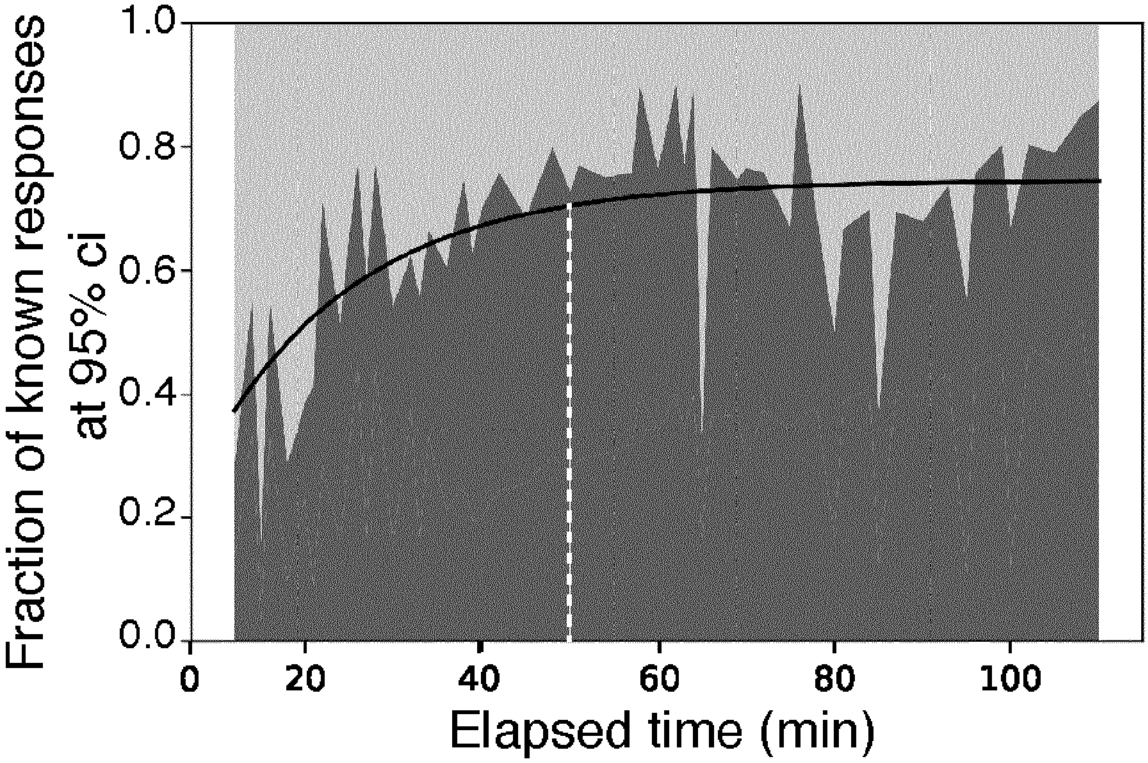


Figure 10

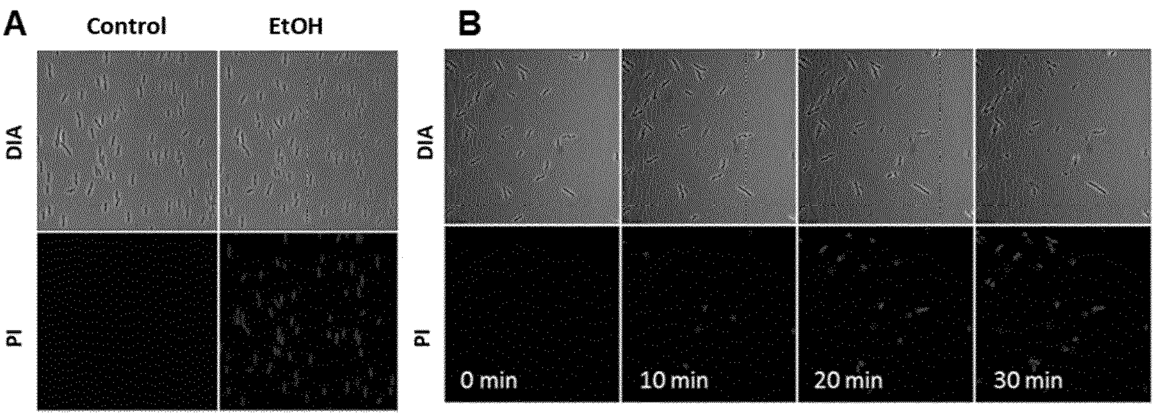


Figures 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200411118 A
- EP 1246650 A
- DE 20215659
- WO 2015042583 A


### Non-patent literature cited in the description


- **CHEN et al.** *Anal. Chem.,* 2010, vol. 82, 1012-1019
- **DAI et al.** *Bioengineering,* 2016, vol. 3, 25
- **CHANTELL.** *Clin. Microbiol. Newsl.,* 2015, vol. 37, 161-167
- **KONG et al.** *Int. J. Food Microbiol.,* 2010, vol. 144, 51-63
- *Carbohydr. Polym.,* 2017, vol. 164, 268-283
- **LIGLER et al.** *Langmuir,* 2001, vol. 17, 5082-5084
- **DUCRET et al.** *Methods Mol. Biol.,* 2013, vol. 966, 97-107
- **SCHATZ C. et al.** *Biomacromolecules,* 2003, vol. 4 (3), 641
- **CATTONI et al.** *PLOS One,* 2013, vol. 8 (10), e76268
- **TANG et al.** *Anal. Chem.,* 2013, vol. 85, 2787-2794
- **HIRAI et al.** *Polym. Bull.,* 1991, vol. 26, 87-94
- **G.G. ALLAN.** *Carbohydrate Research,* 1995, vol. 277 (2), 273
- **VACHOUD et al.** *Carbohydr. Res.,* 1997, vol. 302, 169-177
- **M. DUMONT et al.** *Carbohydrate Polymers,* 2018, vol. 190, 31